(19) Europäisches Patentamt
European Patent Office
Office européen des brevets

(11) EP 2 001 960 B1

# (12) FASCICULE DE BREVET EUROPEEN

(45) Date de publication et mention de la délivrance du brevet:
**05.03.2014 Bulletin 2014/10**

(21) Numéro de dépôt: **07731813.7**

(22) Date de dépôt: **23.03.2007**

(51) Int Cl.:
***C09B 23/14*** *(2006.01)*

(86) Numéro de dépôt international:
**PCT/FR2007/051001**

(87) Numéro de publication internationale:
**WO 2007/110535 (04.10.2007 Gazette 2007/40)**

## (54) COMPOSITION DE TEINTURE COMPRENANT UN COLORANT FLUORESCENT THIOL/ DISULFURE A GROUPE ORTHO-PYRIDINIUM A CHAINE ALKYLENE INTERROMPUE ET A CHARGE CATIONIQUE INTERNE, PROCEDE D'ECLAIRCISSEMENT DES MATIERES KERATINIQUES A PARTIR DE CE COLORANT

FÄRBEMITTEL MIT EINEM THIOL/DISULPHID-FLUORESZENZFARBSTOFF MIT EINER ORTHO-PYRIDINIUMGRUPPE MIT UNTERBROCHENER ALKYLENKETTE UND EINER EXTERNEN KATIONISCHEN LADUNG SOWIE DAVON GEBRAUCH MACHENDES VERFAHREN ZUM AUFHELLEN VON KERATINMATERIALIEN

DYEING COMPOSITION CONTAINING A THIOL/DISULPHIDE FLUORESCENT COLORANT COMPRISING AN ORTHO-PYRIDINIUM GROUP WITH AN INTERRUPTED ALKYLENE CHAIN AND AN EXTERNAL CATIONIC LOAD, AND METHOD FOR LIGHTENING KERATIN MATERIALS USING SAID COLORANT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **24.03.2006 FR 0651035**
**19.04.2006 US 792941 P**
**05.02.2007 FR 0753067**
**12.02.2007 US 900710 P**

(43) Date de publication de la demande:
**17.12.2008 Bulletin 2008/51**

(73) Titulaire: **L'Oréal**
**92665 Asnieres-sur-Seine (FR)**

(72) Inventeurs:
- **GREAVES, Andrew**
**F-77144 Montevrain (FR)**
- **DAUBRESSE, Nicolas**
**F-78170 La Celles St Cloud (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 647 580**
**WO-A-02/078596**
**WO-A-2004/091473**
**WO-A-2005/004822**
**WO-A-2005/097051**
**WO-A-2006/134043**
**US-B1- 6 262 263**

- **OKAWA H ET AL: "SYNTHESIS AND CHARACTERIZATION OF AN ALKANETHIOL THIN FILM CONTAINING A HEMICYANINE DYE" MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, CH, vol. 377, 2002, pages 137-140, XP008056025 ISSN: 1058-725X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne la coloration de matières kératiniques à l'aide de colorants fluorescents thiols/disulfure comprenant un groupe otho-pyridinium et une chaîne alkylène interrompue.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les matières kératiniques telles que les fibres kératiniques, notamment les cheveux, par un système oxydant fort, généralement constitué par du peroxyde d'hydrogène associé ou non à des persels, le plus souvent en milieu alcalin.

Ce système de décoloration présente l'inconvénient de dégrader des matières kératiniques, en particulier les fibres kératiniques, notamment humaines telles que les cheveux et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans les documents FR 2830189 et WO 2004/091473 permet de respecter la qualité de la fibre kératinique lors du traitement mais les colorants fluorescents employés ne présentent pas une résistance aux shampoings satisfaisante.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres kératiniques voir par exemple Journal of the Society of Dyers and Colourists, Guise et Stapleton, 91, 259-264 (1975); Journal of Cosmetic Chemistry, 42, 1-17 (1991); CA 2024509;

De plus il est connu de protéger la ou les fonctions thiols contenues dans une molécule à greffer aux cheveux avant de les appliquer auxdits cheveux WO99/51194. Cependant cette demande ne mentionne pas l'utilisation de colorants fluorescents permettant de colorer ou d'éclaircir des cheveux.

D'autres colorants disulfures connus pour la coloration des fibres kératiniques sont des dérivés disulfures de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un prétraitement réducteur du cheveu. Cependant, ces colorants peuvent occasionner des virages de couleur lors de l'application.

Enfin, le document WO 2005/097051 décrit des colorants disulfures aza-imidazoliums pour la coloration directe de fibres kératiniques.

WO 02/078596 concerne également des compositions pour colorer les fibres kératiniques à partir de colorants diazoïques qui ne sont pas fluorescents. Les demandes de brevet WO 2004/091473 et WO 2005/004822 divulguent des colorants fluorescents para-aminophényl styryle ortho pyridinium à un ou deux chromophores pour colorer les cheveux. Le brevet américain US 6,262,263 B1 s'intéresse à des colorants fluorescents para amino phényl styryle pyridinium substitué en para comme sonde biologique fluorescente.

Par ailleurs, dans l'article scientifique Mol. Cryst. Liq. Cryst., vol. 377, 137-140, il a été décrit la synthèse et la caractérisation de films comprenant des colorants hémicyanines disulfures à chromophore para-aminophényl styryles pyridinium, utilisé pour former des monocouches auto-assemblées (SAM's) sur des surfaces d'or.

Le but de la présente invention est de fournir de nouveaux systèmes de coloration de matières kératiniques en particulier les fibres kératiniques humaines, notamment les cheveux; qui ne présentent pas les inconvénients des procédés de décoloration existants. En particulier, un des buts de la présente invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et

qui n'altèrent pas leurs propriétés cosmétiques.

Ce but est atteint avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent thiol, choisi parmi les colorants de formules **(I)** et **(II)** suivantes :

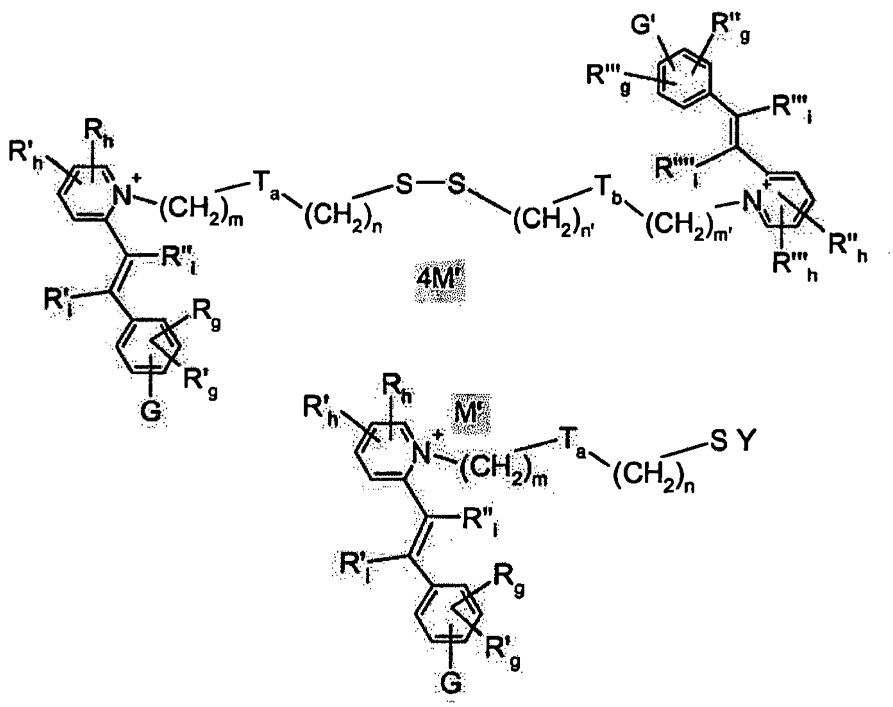

(I)

(II)

leurs sels d'acide organique ou minéral, isomères optiques, isomères géométriques, et les solvates tels que hydrates ; formules **(I)** et **(II)** dans laquelle :

➢ G et G', identiques ou différents, représentent un groupement $-NR_cR_d$ ou $C_1$-$C_6$ alcoxy éventuellement substitué, notamment non substitué ; préférentiellement G, G' représentent un groupement $-NR_cR_d$ ;

➢ $R_c$ et $R_d$ identiques ou différents, représentent un atome d'hydrogène, un groupement aryl($C_1$-$C_4$)alkyle, ou un groupement ($C_1$-$C_6$)alkyle éventuellement substitué ; $R_c$ et $R_d$ représentent particulièrement un atome d'hydrogène ou un groupement ($C_1$-$C_4$)alkyle;

➢ $R_g$, $R'_g$, R"g, $R'''_g$, $R_h$, $R'_h$, $R''_h$ et $R'''_h$, identiques ou différents, représentent un atome d'hydrogène, d'halogène, un groupement amino, (di)($C_1$-$C_4$)alkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonyl-amino, acylamino, carbamoyle , ($C_1$-$C_4$)alkylsulfonylamino, un radical amino-sulfonyle, ou un radical ($C_1$-$C_{16}$)alkyle éventuellement substitué par un groupement choisi parmi ($C_1$-$C_{12}$)alcoxy, hydroxy, cyano, carboxy, amino, (di)($C_1$-$C_4$)alkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ; particulièrement $R_g$, $R'_g$, $R''_g$, $R'''_g$, $R_h$, $R'_h$, $R''_h$ et $R'''_h$ représentent un atome d'hydrogène ou un groupement ($C_1$-$C_4$)alkyle ;

➢ $R'_i$, $R''_i$, $R'''_i$ et $R''''_i$, identiques ou différents, représentent un atome d'hydrogène, ou un groupement ($C_1$-$C_4$) alkyle ; particulièrement $R'_i$, $R''_i$, $R'''_i$ et $R''''_i$, représentent un atome d'hydrogène ;

➢ $T_a$ et $T_b$, identiques ou différents, représentent :

- soit un radical ammonium $-N^+(R)(R°)-$, avec R et R°, identiques ou différents, représentant un atome d'hydrogène, un radical alkyle en $C_1$-$C_4$, hydroxyalkyle en $C_1$-$C_4$ ou un aryl($C_1$-$C_4$)alkyle ;
- soit un radical hétérocycloalkyle ou hétéroaryle, cationique préférentiellement monocycliques, contenant notamment deux hétéroatomes, particulièrement deux atomes d'azote, et comportant notamment de 5 à 7 chaînons tel que l'imidazolium, pyridinium ou pyrrolidinium éventuellement substitué par un groupement ($C_1$-$C_4$) alkyle tel que méthyle ;

particulièrement, $T_a$ et $T_b$ sont identiques, et avantageusement représentent -$N^+$(R)(R°)- ;

➢ m, m', n et n', identiques ou différents, représentent un entier compris inclusivement entre 0 et 6 avec m+n, m'+n' représentent un entier compris inclusivement entre 1 et 10; particulièrement la somme de m+n=m'+n' est un entier compris inclusivement entre 2 et 4 ; préférentiellement m+n=m'+n' est un entier égal à 2 ;

➢ M', identiques ou différents, représentant un contre-ion anionique ; et

➢ Y représente : i) un atome d'hydrogène ; ii) un métal alcalin ; iii) un métal alcalino-terreux; iv) un groupement ammonium : $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe ($C_1$-$C_4$)alkyle ; ou v) un groupement protecteur de fonction thiol ;

étant entendu que :

- lorsque le composé de formule **(I)** ou **(II)** contient d'autres parties cationiques, il se trouve associé à un ou plusieurs contre-ions anioniques permettant d'atteindre l'électroneutralité de la formule **(I)** ou **(II) ;**

leurs sels, isomères optiques, isomères géométriques, et les solvates tels que hydrates.

Un autre objet de l'invention est une composition tinctoriale comprenant, dans un milieu cosmétique approprié au moins un colorant fluorescent de formule (I) ou (II) tels que définis précédemment, et un agent réducteur.

L'invention a aussi pour objet de nouveaux colorants fluorescents de formule **(I)** ou (II) tels que définis précédemment.

Le procédé de coloration selon l'invention permet de colorer de façon visible les matières kératiniques foncées, en particulier les fibres kératiniques humaines foncées, notamment les cheveux foncés.

De plus le procédé de l'invention permet d'obtenir une coloration des matières kératiniques en particulier les fibres kératiniques humaines, notamment les cheveux, sans dégrader ladite matière, rémanente vis-à-vis de shampooings, des agressions courantes (soleil, transpiration), et des autres traitements capillaires. Le procédé de l'invention permet également d'obtenir un éclaircissement des matières kératiniques telles que les fibres kératiniques particulièrement les fibres kératiniques foncées et plus particulièrement les cheveux foncés.

Au sens de l'invention, on entend par matière kératinique foncée celle qui présente une luminescence L* chiffrée dans le système C.I.E. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de « hauteur de ton » avant et après application du composé de formule (I) ou (II).

La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

De préférence, la composition doit, après application sur des cheveux, par exemple châtains, amener aux résultats ci-dessous.

- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :

- les radicaux « aryle » ou « hétéroaryle » ou la partie aryle ou hétéroaryle d'un radical peuvent être substitués par au moins un substituant porté par un atome de carbone, choisi parmi :

  • un radical alkyle en $C_1$-$C_{16}$, de préférence en $C_1$-$C_8$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote;
  • un atome d'halogène tel que chlore, fluor ou brome ;
  • un groupement hydroxy ;
  • un radical alcoxy en $C_1$-$C_2$ ;
  • un radical alkylthio en $C_1$-$C_2$ ;
  • un radical (poly)-hydroxyalcoxy en $C_2$-$C_4$
  • un radical amino ;
  • un radical héterocycloalkyle à 5 ou 6 chaînons ;
  • un radical hétéroaryle à 5 ou 6 chaînons éventuellement cationique, préférentiellement imidazolium, et éventuellement substitué par un radical ($C_1$-$C_4$) alkyle, préférentiellement méthyle ;
  • un radical amino substitué par un ou deux radicaux alkyle, identiques ou différents, en $C_1$-$C_6$ éventuellement porteurs d'au moins :

    i) un groupement hydroxy,
    ii) un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,

  • -NR-COR' dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
  • $(R)_2N$-CO- dans lequel les radicaux R, identiques ou non, représentent un atome d'hydrogène, un radical alkyle en $C_1$-$C_4$ éventuellement porteur d'au moins un groupement hydroxy ;
  • $R'SO_2$-NR- dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ;
  • $(R)_2N$-$SO_2$- dans lequel les radicaux R, identiques ou non, représentent un atome d'hydrogène, un radical alkyle en $C_1$-$C_4$ éventuellement porteur d'au moins un groupement hydroxy,
  • un radical carboxylique sous forme acide ou salifiée (de préférence avec un métal alcalin ou un ammonium, substitué ou non) ;
  • un groupement cyano ;
  • un groupement polyhalogénoalkyle comprenant de 1 à 6 atomes de carbones et de 1 à 6 atome d'halogène, identiques ou différents, le groupement polyhalogénoalkyle est par exemple le trifluorométhyle ;

- la partie cyclique ou hétérocyclique d'un radical non aromatique peut être substituée par au moins un substituant porté par un atome de carbone choisi parmi les groupements :

  • hydroxy,
  • alcoxy en $C_1$-$C_4$,
  • (poly)hydroxyalcoxy en $C_2$-$C_4$,
  • un radical alkylthio en $C_1$-$C_2$ ;
  • RCO-NR'- dans lequel le radical R' est un atome d'hydrogène, un radical alkyle en $C_1$-$C_4$ éventuellement porteur d'au moins un groupement hydroxy et le radical R est un radical alkyle en $C_1$-$C_2$, amino substitué par deux groupements alkyle identiques ou différents en $C_1$-$C_4$ éventuellement porteurs d'au moins un groupement hydroxy ;

- RCO-O- dans lequel le radical R est un radical alkyle en $C_1$-$C_4$, amino substitué par un ou deux groupements alkyle identiques ou différents en $C_1$-$C_4$ éventuellement porteurs d'au moins un groupement hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- RO-CO- dans lequel le radical R est un radical alkyle en $C_1$-$C_4$ éventuellement porteurs d'au moins un groupement hydroxy ;

- un radical cyclique, hétérocyclique, ou une partie non aromatique d'un radical aryle ou hétéroaryle, peut également être substitué par un ou plusieurs groupements oxo ou thioxo ;
- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un radical « diarylalkyle » représente un groupement comportant sur le même atome de carbone d'un groupement alkyle deux groupement aryle, identiques ou différents tel que diphénylméthyle ou 1,1-diphényléthyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un radical « dihétéroarylalkyle » représente un groupement comportant sur le même atome de carbone d'un groupement alkyle deux groupement hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1,3,5-trimétylsillylphényle et l'adamantyle ;
- un « radical hétérocyclique ou hétérocycle» est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en $C_1$-$C_{16}$, linéaire ou ramifié, de préférence en $C_1$-$C_8$ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en $C_1$-$C_4$, iii) acylamino, iv) amino éventuellement substitué par un ou deux radicaux alkyle, identiques ou différents, en $C_1$-$C_4$, lesdits radicaux alkyle pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire $-N^+R'R''R'''$, M pour lequel R', R", R"', identiques ou différents représentent un atome d'hydrogène, ou un groupement alkyle en $C_1$-$C_4$, ou alors $-N^+R'R''R'''$ forme un hétéroaryle tel que imidazolium éventuellement substitué par un groupement $C_1$-$C_4$ alkyle, et $M^-$ représente le contre-ion de l'acide organique, minéral ou de l'halogénure correspondant,
- un « radical alcoxy » est un radical alkyle-oxy ou alkyl-O- pour lequel le radical alkyle est un radical hydrocarboné, linéaire ou ramifié, en $C_1$-$C_{16}$ préférentiellement en $C_1$-$C_8$ ;
- un « radical alkylthio » est un radical alkyl-S- pour lequel le radical alkyle est un radical hydrocarboné, linéaire ou ramifié, en $C_1$-$C_{16}$ préférentiellement en $C_1$-$C_8$ ; lorsque le groupement alkylthio est éventuellement substitué, cela sous entend que le groupe alkyle est éventuellement substitué tel que défini précédemment ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-$S(O)_2OH$ tels que d'acide méthylsulfonique et d'acide éthylsulfonique ; v) d'acides arylsulfoniques : Ar-$S(O)_2OH$ tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-O-S(O)OH tels que d'acide méthoxysulfinique et d'acide

éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3COOH$ ; xiv) d'acide triflique $CF_3SO_3H$ et xv) d'acide tétrafluoroborique $HBF_4$ ;

- un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les $C_1$-$C_6$ alkylsulfonates : Alk-$S(O)_2O^-$ tels que le méthylsulfonate ou mésylate et l'éthylsulfonate ; iv) les arylsulfonates : Ar-$S(O)_2O^-$ tel que le benzènesulfonate et le toluènesulfonate ou tosylate ; v) le citrate ; vi) le succinate ; vii) le tartrate ; viii) le lactate ; ix) les alkylsulfites : Alk-O-$S(O)O^-$ tels que le méthysulfite et l'éthylsulfite ; x) les arylsulfites : Ar-O-$S(O)O^-$ tels que le benzènesulfite et le toluènesulfite ; xi) les alkylsulfates : Alk-O-$S(O)_2O^-$ tel que le méthyl sulfate et l'éthylsulfate ; xii) les arylsulfates : Ar-O-$S(O)_2O^-$, xiii) le phosphate ; xiv) l'acétate ; xv) le triflate ; et xvi) les borates tels que le tétrafluoroborate

Les colorants fluorescents de formule (I) ou (II) sont des composés capables d'absorber dans le rayonnement UV ou visible à une longueur d'onde $\lambda_{abs}$ comprise entre 250 et 800 nm et capables de réémettrent dans le domaine du visible à une longueur d'onde d'émission $\lambda_{ém}$ comprise entre 400 et 800 nm.

De préférence les composés fluorescents de l'invention sont des colorants capables d'absorber dans le visible $\lambda_{abs}$ comprise entre 400 et 800 nm et de réémettre dans le visible $\lambda_{ém}$ comprise entre 400 et 800 nm. Plus préférentiellement les colorants fluorescents de formule (I) ou (II) sont des colorants capables d'absorber à une $\lambda_{abs}$ comprise entre 420 nm et 550 nm et de réémettre dans le visible à une $\lambda_{ém}$ comprise entre 470 et 600 nm.

Les composés fluorescents de l'invention qui contiennent une fonction SY de formule (II) peut se trouver sous la forme covalente -S-Y ou ionique -$S^-$ $Y^+$ selon la nature de Y et du pH du milieu.

Un mode particulier concerne les colorants fluorescents thiols de formule (II) à fonction SY où Y représente un atome d'hydrogène, ou un métal alcalin. Avantageusement Y représente un atome d'hydrogène.

Conformément à un autre mode de réalisation particulier de l'invention, dans la formule (II) précitée, Y est un groupement protecteur connu par l'homme du métier comme par exemple ceux décrits dans les ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Particulièrement lorsque Y représente un groupement protecteur de la fonction thiol, Y est choisi parmi les radicaux suivants :

■ ($C_1$-$C_4$)alkylcarbonyle,
■ ($C_1$-$C_4$)alkylthiocarbonyle,
■ ($C_1$-$C_4$)alcoxycarbonyle,
■ ($C_1$-$C_4$)alcoxythiocarbonyle,
■ ($C_1$-$C_4$)alkylthio-thiocarbonyle,
■ (di) ($C_1$-$C_4$) (alkyl)aminocarbonyle,
■ (di) ($C_1$-$C_4$) (alkyl)aminothiocarbonyle,
■ arylcarbonyle comme phénylcarbonyle ;
■ aryloxycarbonyle ;
■ aryl($C_1$-$C_4$)alcoxycarbonyle ;
■ (di) ($C_1$-$C_4$) (alkyl)aminocarbonyle comme diméthylaminocarbonyle;
■ ($C_1$-$C_4$)(alkyl)arylaminocarbonyle
■ carboxy;
■ $SO_3^-$ ; $M^+$ avec $M^+$ représentant un métal alcalin tel que le sodium ou le potassium ou alors $M^+$ et M' de la formule (II) sont absents ;
■ aryle éventuellement substitué tel que le phényle, dibenzosubéryle, ou 1,3,5-cycloheptatriényle,
■ hétéroaryle éventuellement substitué ; dont notamment l'hétéroaryle cationiques ou non, comprenant de 1 à 4 hétéroatomes suivants :

i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;

ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par

un ou plusieurs groupements tels que ($C_1$-$C_4$)alkyle comme méthyle, ou polyhalogéno($C_1$-$C_4$)alkyle comme trifluorométhyle ;

iii) ou tricyclique ABC suivant :

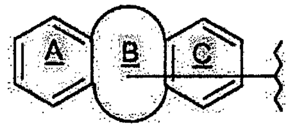

dans lequel les deux cycles A C comportent éventuellement un hétéroatome, et le cycle B est un cycle à 5, 6 ou 7 chaînons particulièrement à 6 chaînons et contient au moins un hétéroatome comme pypéridyle, pyranyle ;

■ hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme ($C_1$-$C_4$) alkyle, oxo ou thioxo ; ou l'hétérocycle représente le groupement suivant :

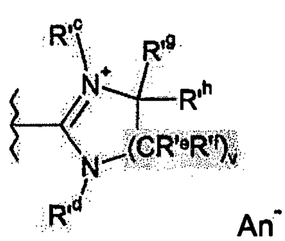

An⁻

dans lequel $R'^c$, $R'^d$, $R'^e$, $R'^f$, $R'^g$ et $R'^h$, identiques ou différents représentent un atome d'hydrogène ou un groupement ($C_1$-$C_4$) alkyle, ou alors deux groupement $R'^g$ avec $R'^h$, et/ou $R'^e$ avec $R'^f$ forment un groupement oxo ou thioxo, ou alors $R'^g$ avec $R'^e$ forment ensemble un cycloalkyle ; et v représente un entier compris inclusivement entre 1 et 3 ; préférentiellement $R'^c$ à $R'^h$ représentent un atome d'hydrogène ; et $An^-$ représente un contre-ion ;

■ isothiouronium $-C(NR'^cR'^d)=N^+R'^eR'^f$; $An^-$ avec $R'^c$, $R'^d$, $R'^e$ et $R'^f$, identiques ou différents représentent un atome d'hydrogène ou un groupement ($C_1$-$C_4$)alkyle ; préférentiellement $R'^c$ à $R'^f$ représentent un atome d'hydrogène ; et $An^-$ représente un contre-ion ;

■ isothiourée $-C(NR'^cR'^d)=NR'^e$; avec $R'^c$, $R'^d$, $R'^e$, tels que définis précédemment ;

■ (di)aryl($C_1$-$C_4$)alkyle éventuellement substitué tel que le 9-anthracénylméthyle, phénylméthyle ou diphénylméthyle éventuellement substitué par un plusieurs groupements notamment choisis parmi ($C_1$-$C_4$) alkyle, ($C_1$-$C_4$) alcoxy comme le méthoxy, hydroxy, ($C_1$-$C_4$) alkylcarbonyle, (di) ($C_1$-$C_4$) (alkyl)amino comme le diméthylamino ;

■ (di)hétéroaryl($C_1$-$C_4$)akyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que alkyle particulièrement méthyle, avantageusement le (di)hétéroaryl($C_1$-$C_4$)akyle est (di)hétéroarylméthyle ou (di) hétéroaryléthyle ;

■ $CR^1R^2R^3$ avec $R^1$, $R^2$ et $R^3$ identiques ou différents, représentant un atome d'halogène ou un groupe choisi parmi :

- ($C_1$-$C_4$) alkyle ;
- ($C_1$-$C_4$) alcoxy ;
- aryle éventuellement substitué tel que phényle éventuellement substitué par un ou plusieurs groupements comme ($C_1$-$C_4$)alkyle, ($C_1$-$C_4$)alcoxy, hydroxy ;
- hétéroaryle éventuellement substitué tel que thiophényle, furanyle, pyrrolyle, pyranyle, pyridyle, éventuellement substitué par un groupement ($C_1$-$C_4$)alkyle ;
- $P(Z^1)R'^1R'^2R'^3$ avec $R'^1$, et $R'^2$ identiques ou différents représentent un groupement hydroxy, ($C_1$-$C_4$)alcoxy ou alkyle, $R'^3$ représente un groupement hydroxy ou ($C_1$-$C_4$)alcoxy, et $Z^1$ représente un atome d'oxygène ou de soufre ;

■ cyclique stériquement encombré tel que le groupe adamantyle ; et alcoxy($C_1$-$C_4$)alkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule (II) comportent un groupement Y i) hétéroaryle monocyclique à 5 ou 6 chaînons aromatiques, cationiques comprenant de 1 à 4 hétéroatomes choisis parmi l'oxygène, le soufre et l'azote, tels que oxazolium,, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme méthyle, ou polyhalogéno($C_1$-$C_4$)alkyle comme trifluorométhyle ; iii) ou hétérocyclique suivant :

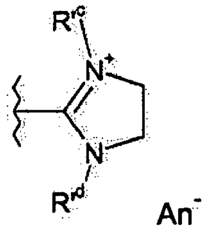

dans lequel R'c et R'd, identiques ou différents, représentent un atome d'hydrogène ou un groupement ($C_1$-$C_4$)alkyle ; préférentiellement R'c à R'd représentent un groupement ($C_1$-$C_4$)alkyle tel que méthyle ; et $An^-$ représente un contre-ion.

Particulièrement Y représente un groupement choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupements étant éventuellement substitués par un ou plusieurs groupes ($C_1$-$C_4$) alkyle notamment méthyle.

En particulier Y représente un métal alcalin ou un groupement protecteur tel que:

- ➢ ($C_1$-$C_4$)alkylcarbonyle comme méthylcarbonyle ou éthylcarbonyle ;
- ➢ arylcarbonyle comme phénylcarbonyle ;
- ➢ ($C_1$-$C_4$)alcoxycarbonyle ;
- ➢ aryloxycarbonyle ;
- ➢ aryl($C_1$-$C_4$)alcoxycarbonyle ;
- ➢ (di) ($C_1$-$C_4$) (alkyl)aminocarbonyle comme diméthylaminocarbonyle;
- ➢ ($C_1$-$C_4$)(alkyl)arylaminocarbonyle ;
- ➢ aryle éventuellement substitué tel que le phényle ;
- ➢ hétéroaryle monocyclique à 5 ou 6 chaînons tels que imidazolyle ou pyridyle
- ➢ hétéroaryle monocyclique cationique à 5, 6 chaînons tels que pyrylium, pyridinium, pyrimidinium, pyrazinium, pyridazinium, triazinium, imidazolium ; ces groupements étant éventuellement substitué par un ou plusieurs, identiques ou différents, groupes ($C_1$-$C_4$)alkyle tel que méthyle;
- ➢ hétéroaryle bicyclique cationique à 8 à 11 chaînons tels que benzoimidazolium, ou le benzoxazolium ; ces groupements étant éventuellement substitué par un ou plusieurs, identiques ou différents, groupes ($C_1$-$C_4$)alkyle tel que méthyle ;
- ➢ hétérocycle cationique de formule suivante :

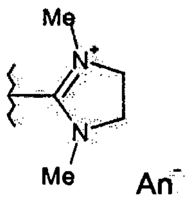

- ➢ isothiouronium $-C(NH_2)=N^+H_2$; $An^-$;
- ➢ isothiourée $-C(NH_2)=NH$ ;
- ➢ $SO_3^-$, $M^+$ avec $M^+$ représentant un métal alcalin tel que le sodium ou le potassium ou alors $M^+$ et M' de la formule (II) sont absents.

A titre d'exemple de colorants fluorescent de formule (I) ou (II) on peut citer notamment le composé suivant: le

| | |
|---|---|
| 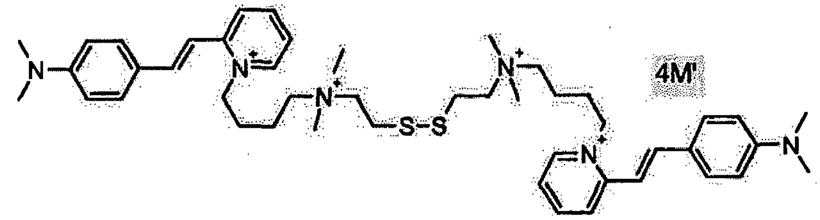 | |
| 1 | |
| 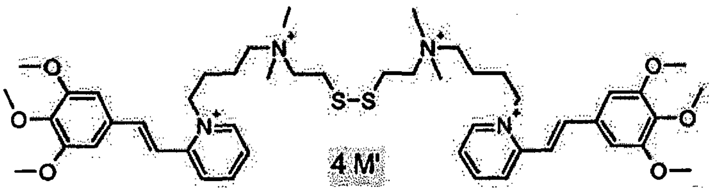 | |
| 2 | |
| 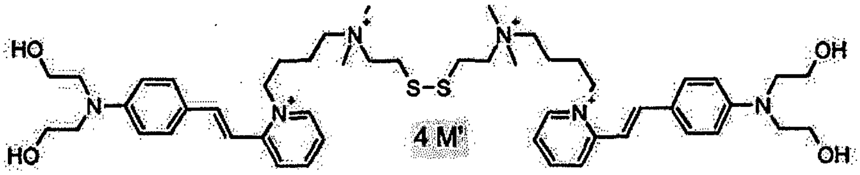 | |
| 3 | |
| 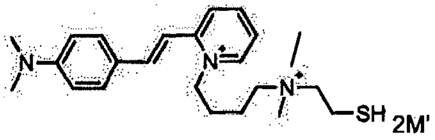 | 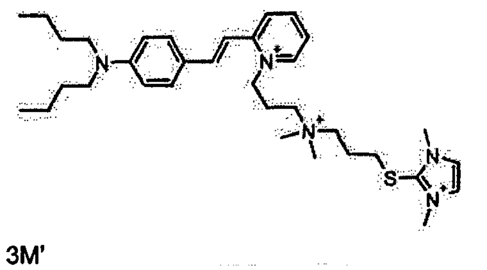 |
| 4 | 5 |
| 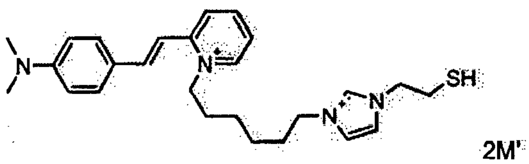 | 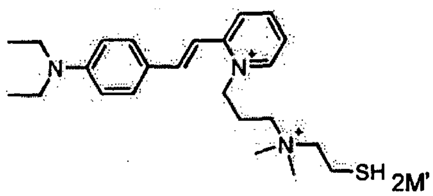 |
| 6 | 7 |
| 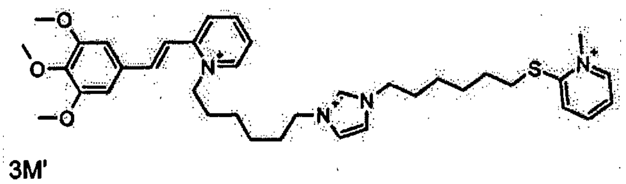 | 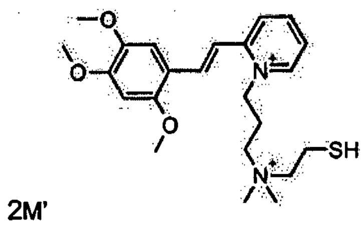 |
| 8 | 9 |

(suite)

| |
|---|
| 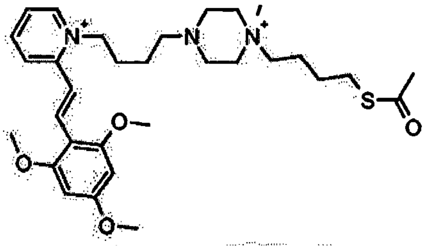 2M' |
| 10 |
| 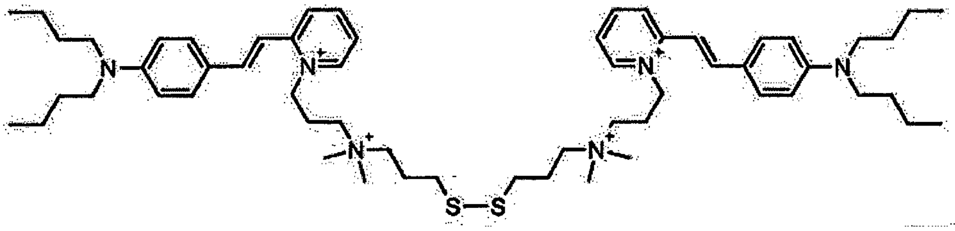 4M' |
| 11 |
| 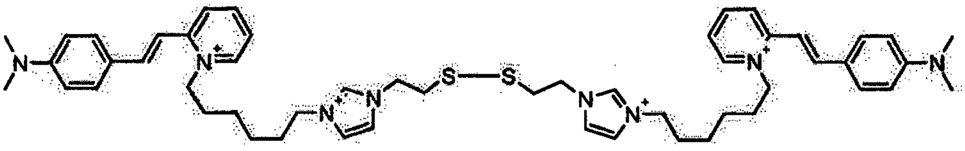 4M' |
| 12 |
| 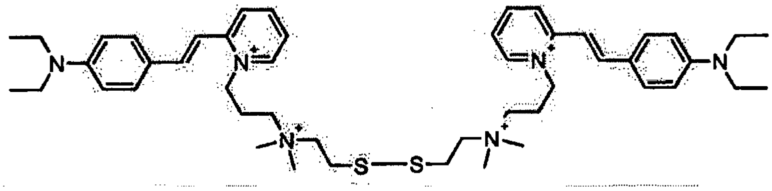 4M' |
| 13 |
| 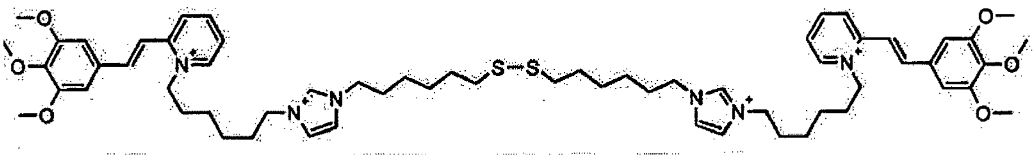 4M' |
| 14 |
| 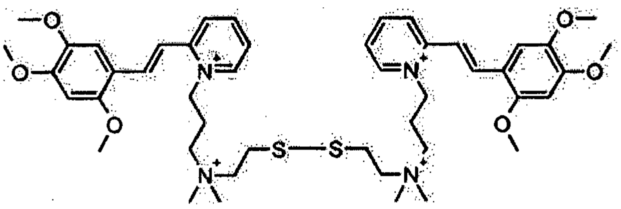 4M' |
| 15 |

(suite)

| 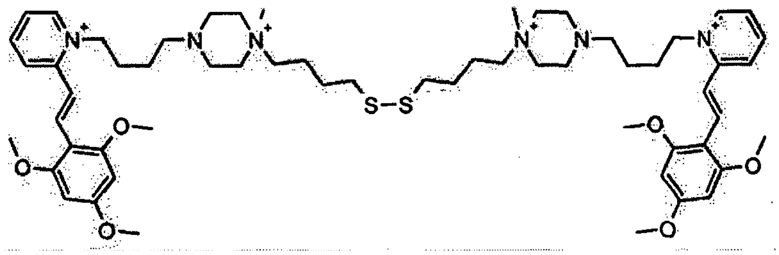 4M' |
|---|
| 16 |

Avec M', identiques ou différents, représentant un contre-ion anionique.

Les colorants thiols protégés de formule (II") peuvent être synthétisés en deux étapes. La première étape consistant à préparer le colorant thiol non protégé (II') selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule (II"). A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Nous pouvons illustrer cette méthode par la méthode consistant i) à générer des colorants fluorescents thiols de formule (II-H) par réduction d'un colorant fluorescent à deux chromophores, portant une fonction disulfure -S-S- tels que (I') et ii) à protéger selon les méthodes classiques ladite fonction thiol de (II-H) avec le réactif 7 Y'R pour accéder au colorants fluorescents thiols protégés de formule (II'). Le composé thiol (II-H) peut également être métallé avec un métal alcalin ou alcalino terreux Met* pour conduire au colorant fluorescent thiolate de formule (II").

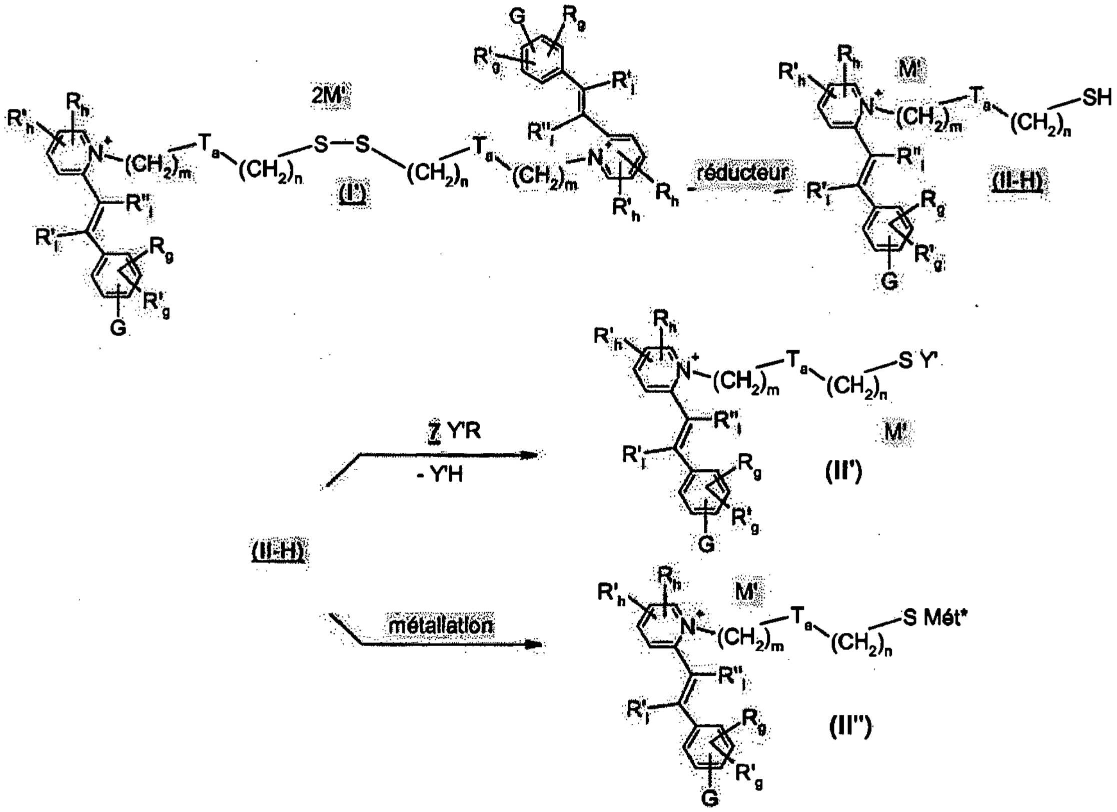


avec Y' représentant un groupement protecteur de fonction thiol ; Met* représentant un métal alcalin ou un métal alcalino térreux, particulièrement le sodium ou le potassium, étant entendu que lorsque le métal est un métal alkalino-terreux 2 chromophores à fonction thiolate-$S^-$ peuvent être associés à 1 Métal$^{2+}$.

Avec $R_g$, $R'_g$, $R_h$, $R'_h$, $R_l$, $R'_l$, m, n, G, $T_a$ et M' sont tels que définis précédemment ; Y' représente un groupement protecteur de fonction thiol ; R représente un groupe partant nucléofuge, comme par exemple mésylate, tosylate, triflate ou halogénure.

Selon une autre possibilité, on peut faire réagir un composé thiol protégé (b) par un groupement protecteur Y' tel que défini précédemment préparé selon une des procédures décrites dans les ouvrages cités précédemment, ledit composé thiol protégé comprenant au moins une fonction nucléophile avec une quantité suffisante, préférentiellement équimolaire, d'un "chromophore fluorescent réactif' ou d'un composé comprenant un tel "chromophore fluorescent réactif" (a). En d'autres termes (a) comprend une fonction électrophile pour former une liaison covalente Σ comme cela peut être schématisé ci-dessous dans la préparation de colorants fluorescents de formule (II'''):

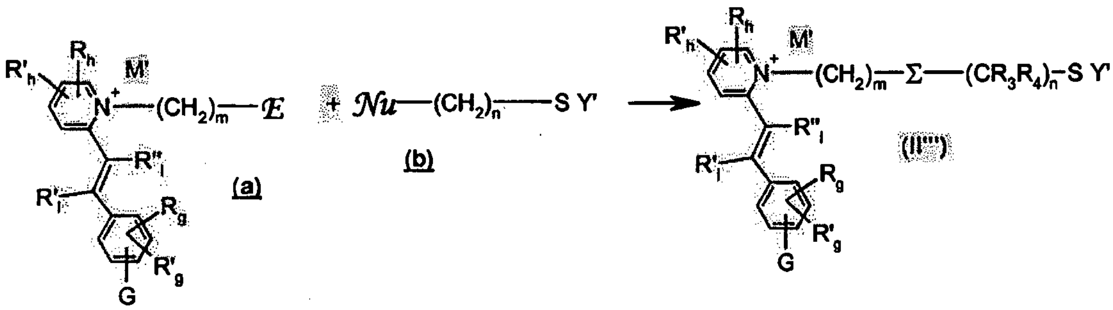


avec $R_g$, $R'_g$, $R_h$, $R'_h$, $R_l$, $R'_l$, m, n, G, et Y' et M' sont tels que définis précédemment ; $\mathcal{N}u$ représentant un groupement nucléophile ; $\mathcal{E}$ représentant un groupement électrophile ; et Σ la liaison générée après attaque du nucléophile sur l'électrophile.

A titre d'exemple, les liaisons covalentes Σ pouvant être générées sont répertoriées dans le tableau ci-dessous à partir de condensation d'électrophiles avec des nucléophiles :

| Electrophile $\mathcal{E}$ | Nucléophile $\mathcal{N}u$ | Liaisons covalentes Σ |
|---|---|---|
| Halogène (Hal), | Amines : N(R)(R°) | $-N^+(R)(R°)-$ (contre ion : $Hal^-$) |
| Halogène (Hal) | hétérocycloalkyle | Hétérocycloalkyle cationique (contre ion : $Hal^-$) |
| Halogéne (Hal) | hétéroaryle | hétéroaryle cationique (contre ion : $Hal^-$) |
| Alcoxy (-Oalkyle) | Amines : N(R)(R°) | $-N^+R)(R°)-$(contre ion : $AlkyleO^-$) |
| Aryloxy (-Oaryle) | Amines : N(R)(R°) | $-N^+(R)(R°)-$ (contre ion : $AryleO^-$) |
| Alkylsulfate ($-OS(O)_2$alkyle) | Amines : N(R)(R°) | $-N^+(R)(R°)-$ (contre ion : alkyle-$SO_3^-$) |
| Arylsulfate (($-OS(O)_2$aryle) | Amines : N(R)(R°) | $-N^+(R)(R°)-$(contre ion : aryle-$SO_3^-$) |

On pourra également utiliser un réactif thiol Y'-SH comprenant un groupement Y' tel que défini précédemment dont la fonction nucléophile SH peut réagir sur l'atome de carbone en alpha de l'atome d'halogène porté par le chromophore fluorescent (a) pour conduire au colorant fluorescent thiol protégé de formule (II'):

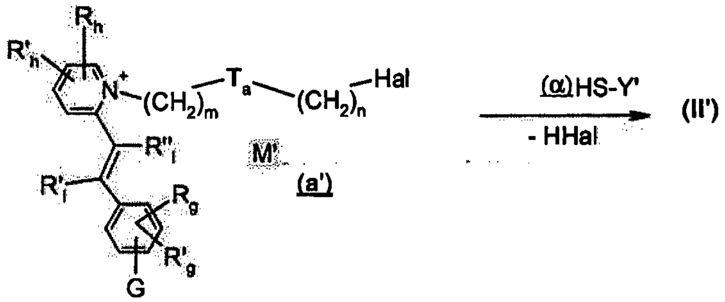


avec $R_g$, $R'_g$, $R_h$, $R'_h$, $R_l$, $R'_l$, $T_a$, m, n, G, Y', (II') et M' sont tels que définis précédemment, et Hal représentant un atome d'halogène nucléofuge tel que le brome, l'iode ou le chlore.

Plus particulièrement on pourra substituer un groupement partant nucléofuge par un groupement thiourée (S=C(NRR)NRR) pour générer les isothiouroniums. Par exemple si le groupement thiourée est une thioimidazolinium (β), le schéma réactionnel est le suivant :

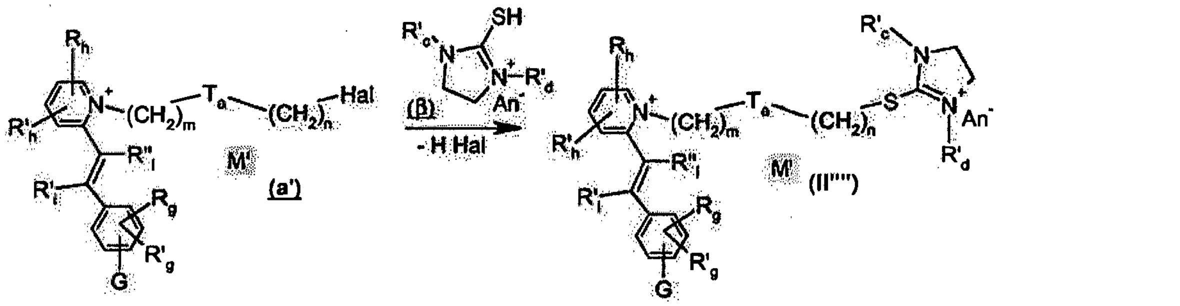


avec $R_g$, $R'_g$, $R_h$, $R'_h$, $R_l$, $R'_l$, $R'_c$, $R'_d$, $T_a$, $An^-$, m, n, G, et M' sont tels que définis précédemment.

Selon une autre variante il est possible de générer un intermédiaire imidazoline à partir de l'halogénure comprenant le chromophore fluorescent (a') et une thioimidazoline (b') pour conduire après alkylation par un réactif R-Gp avec R représentant un groupement alkyle et Gp un groupe partant tel qu'un halogène comme le chlore, brome, iode, ou un groupement mésylate ou tosylate, au colorant fluorescent de formule (II'''').

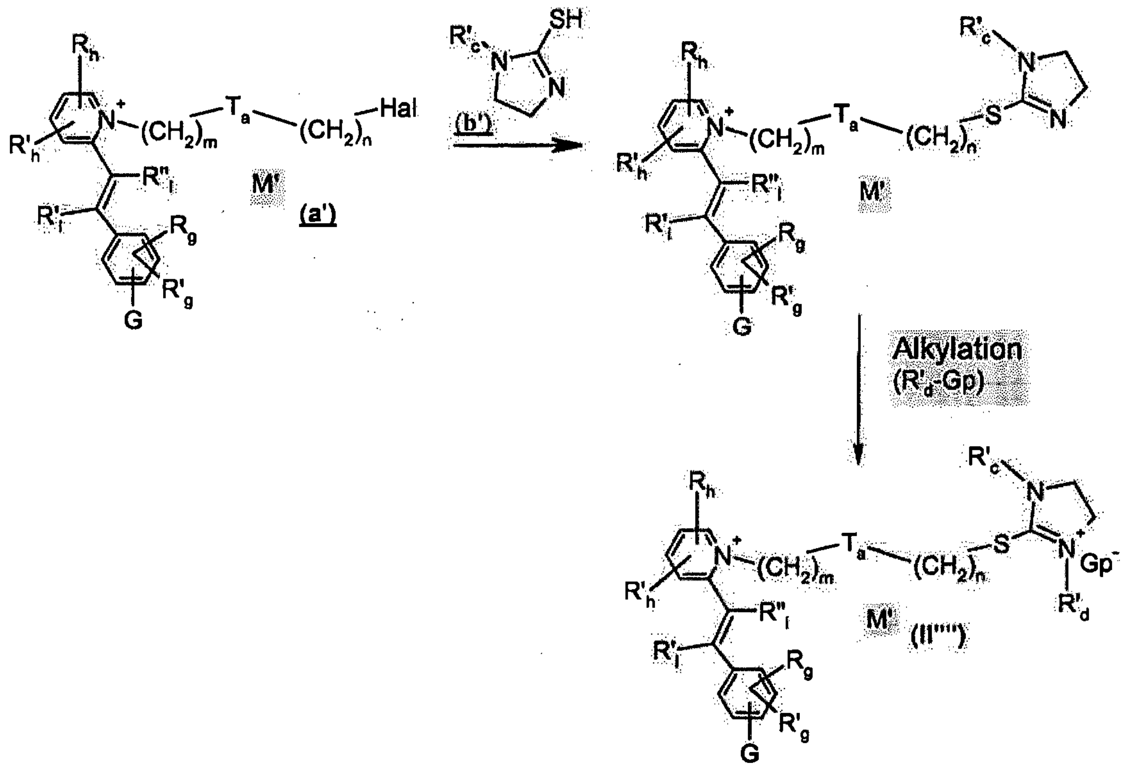


Une variante est d'utiliser à la place de l'halogénure comprenant le chromophore fluorescent (a) un chromophore comprenant un autre type de nucléofuge tel que le tosylate, mésylate.

Conformément à une autre possibilité, certains colorants fluorescents thiols protégés (II''') peuvent être obtenus en faisant réagir un composé thiol protégé, avec un composé portant deux fonctions acide carboxylique activées selon les méthodes classiques (par exemple réaction avec un carbodiimide ou avec le chlorure de thionyle). Le produit résultant (d) est ensuite mis à réagir avec un chromophore fluorescent porteur d'une fonction nucléophile (c), par exemple de type amine primaire ou secondaire, ou de type alcool aliphatique.

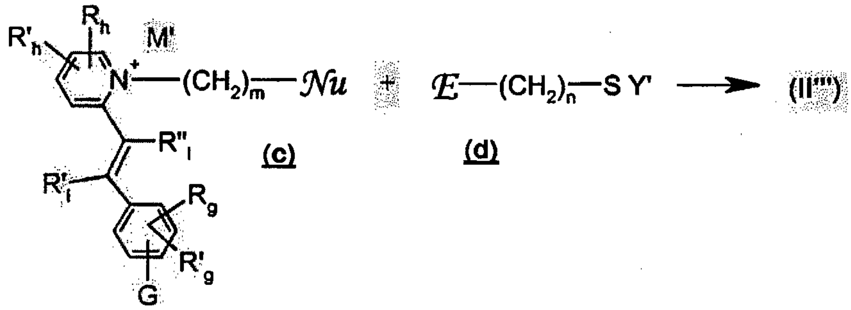

avec $R_g$, $R'_g$, $R_h$, $R'_h$, $R_l$, $R'_l$, $R'_e$, $R'_d$, $An^-$, m, n, G, M', *E*, *Nu* et (II''') tels que définis précédemment.

Une variante de synthèse est de combiner à la première voie la voie précédente ie à partir de deux équivalents du réactif nucléophile (c) avec un réactif diéléctrophile disulfure (l) il est possible de générer après condensation le produit dichromophorique disulfure (I''), ce dernier pouvant subir une réduction pour former le colorant thiol fluorescent hétérocyclique qui a son tour peut soit être protéger pour former le colorant fluorescent thiol protégé (II''') ou soit être métallé par un métal alcalin pour conduire au colorant fluorescent thiol hétérocyclique métallé ($II'''_{Métal}$) :

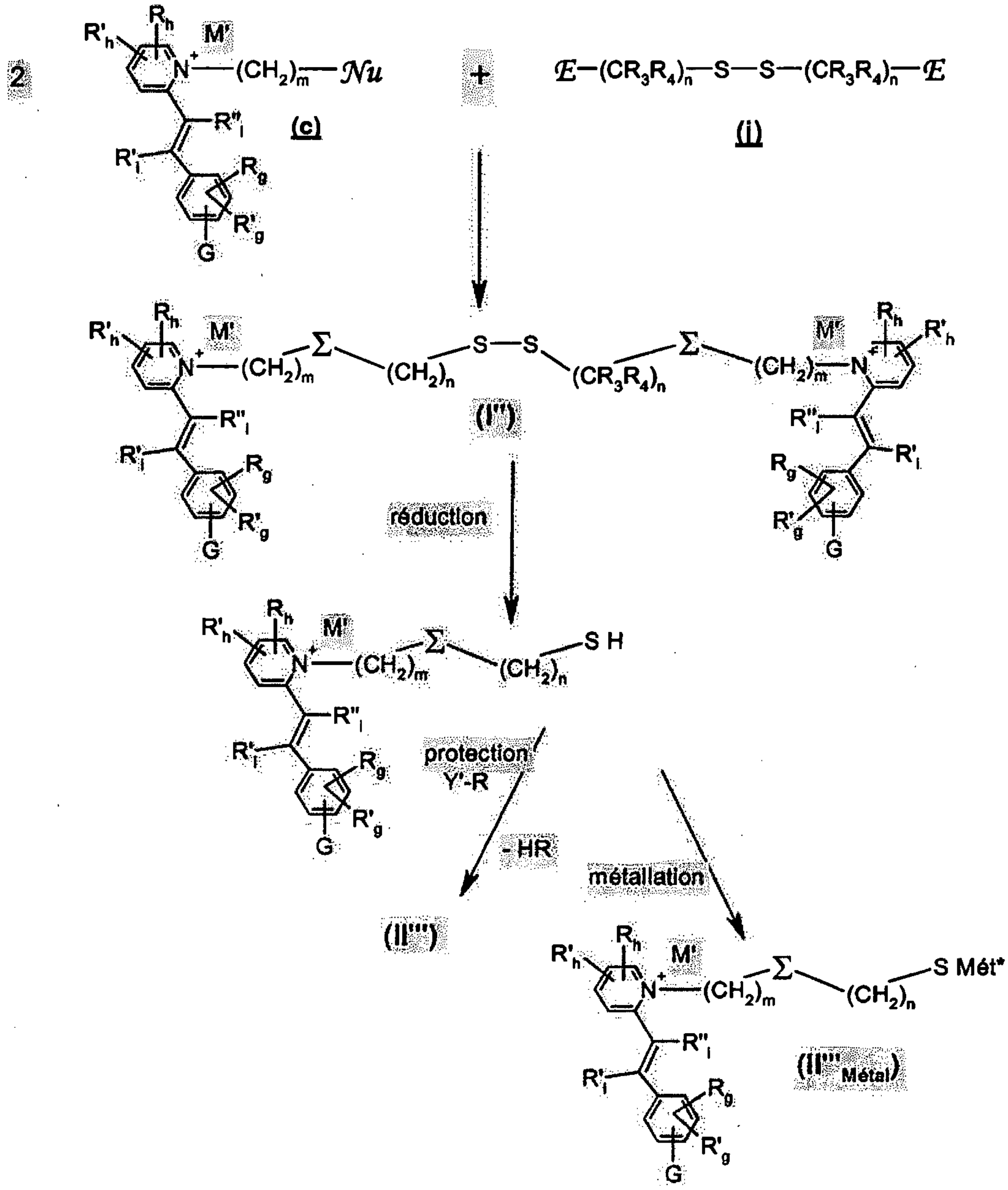


Conformément à une autre possibilité, les colorants fluorescents thiols protégés de formule (II''') peuvent être obtenus par réaction d'un composé comprenant un groupement thiol protégé par un groupement Y', et un groupement hydroxy activé préalablement en groupement partant nucléofuge <u>(d')</u>, comme par exemple mésylate, tosylate, triflate ou halogénure avec un chromophore styrylpyridine <u>(c')</u>.

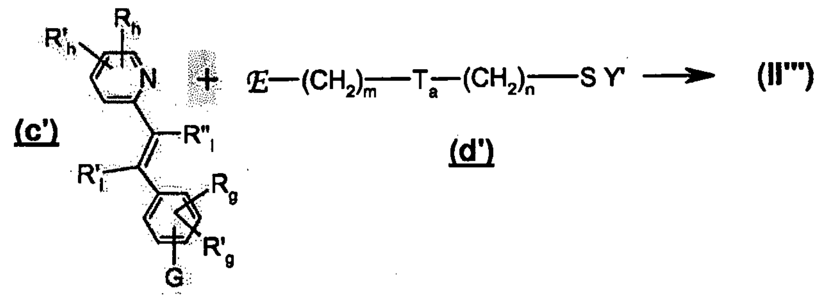

Avec $R_g$, $R'_g$, $R_h$, $R'_h$, $R_l$, $R'_l$, $An^-$, $T_a$, m, n, G, M', 𝕰, et (II''') sont tels que définis précédemment.

A titre d'exemple un composé contenant un groupement thiol protégé, contient un groupement partant nucléofuge R, comme par exemple mésylate, tosylate, ou triflate qui peut subir l'attaque nucléophile de l'amine portée par le chromophore fluorescent styrylique :

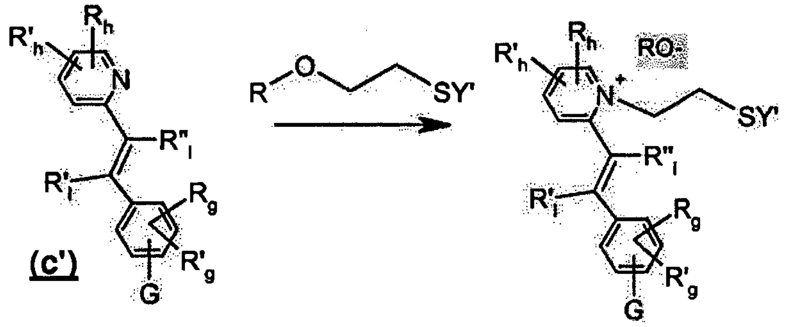

Une autre alternative provient de l'utilisation d'halogénures comme groupement partant nucléofuge sur un composé thiol pouvant être substitué par un fonction amine primaire par exemple portée par un chromophore fluorescent styrylique :

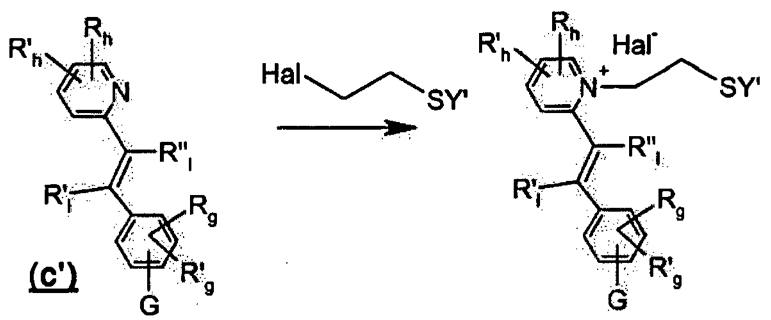

Conformément à une autre possibilité, les colorants fluorescents thiols de formule (II) selon l'invention peuvent être obtenus par réaction d'un composé comprenant un groupement thiol Y tel que défini précédemment et un groupement électrophile (f) avec un composé pyridinium comprenant un groupement nucléophile. A titre d'exemple, on pourra condenser un aldéhyde, cétone ou un thioaldéhyde, thiocétone lorsque G' représente un atome d'oxygène ou un soufre avec un « méthylène activé » tel que l'alkylpyridinium (e) pour générer un liaison éthylène >C=C<. Cette réaction est communément appelée condensation de « Knoevenagel ». Par « méthylènes activés » on sous entend ceux qui comporte préférentiellement en position 2 ou 4 du groupement pyridinium un groupement méthylène $R_1$-$CH_2$-:

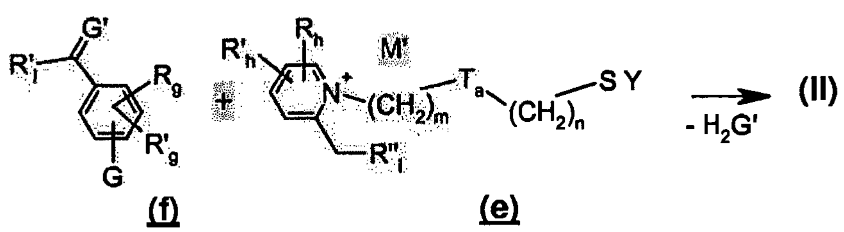


avec $R_g$, $R'_g$, $R_h$, $R'_h$, $R_l$, $R'_l$, $T_a$, G, G', m, n, Y et M' tels que définis précédemment et G représentant un atome d'oxygène, de soufre, ou un groupement $NR^a$ avec $R^a$ représentant un atome d'hydrogène ou une groupement alkyle.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupements protecteurs classiques. Les colorants fluorescents thiols

sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par les méthodes classiques connues par l'homme du métier. A titre d'exemple il est possible de synthétiser le réactif (I') à partir de 2 équivalents de dérivé pyridinique 1 et un équivalent de réactif disulfure comprenant deux groupes partants Gp, pour conduire au sel de dipyridinium disulfure 3 qui peut se condenser à son tour avec deux équivalents de composé f pour conduire à (I').

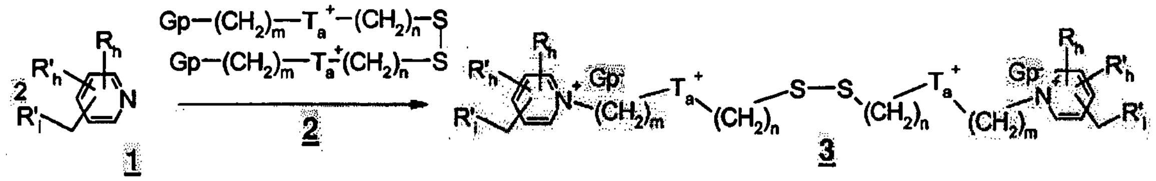


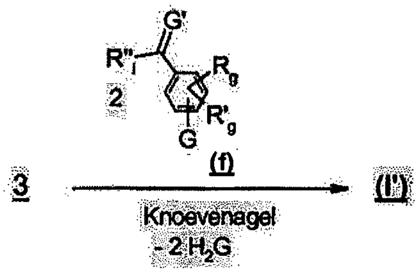


avec Gp représentant un groupe partant nucléofuge, comme par exemple mésylate, tosylate, triflate ou halogénure. Les contre-ions $Gp^-$ des composés (I'), ci-dessus peuvent être remplacés par des contre-ions M' d'autres natures à partir de méthodes connues par l'homme du métier notamment par résine échangeuse d'ion.

Les colorants disulfures dissymétriques de formule (I) peuvent être synthétisés en une étape en faisant réagir un colorant fluorescent thiol non protégé avec un colorant fluorescent thiol protégé par Y' pour former le colorant disulfure de formule (I).

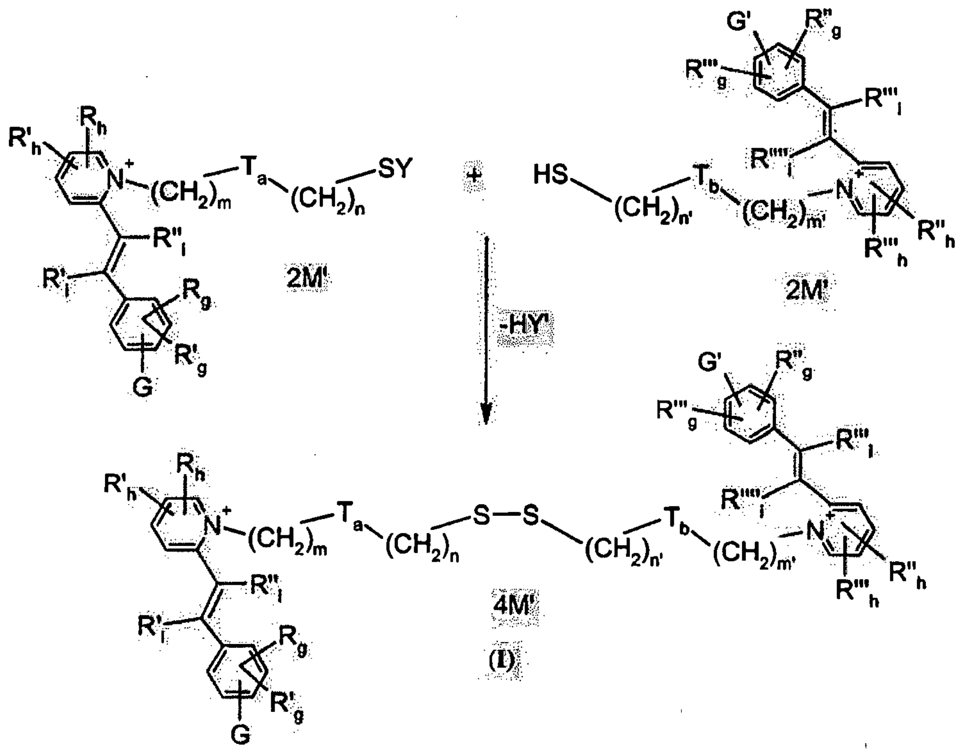

avec $R_g$, $R'_g$, $R''_g$, $R'''_g$, $R_h$, $R'_h$, $R''_h$. $R'''_h$, $R_l$, $R'_l$, $R''_l$, $R'''_l$, G, G', m, m', n, n', $T_a$, $T_b$, et M' sont tels que définis précédemment ; Y' représente un groupement protecteur de fonction thiol.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupements protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

La composition de l'invention contient au moins un colorant fluorescent de formule (I) ou (II). Outre la présence d'au moins un colorant fluorescent de formule (I) ou (II), la composition de l'invention peut également contenir un agent réducteur. Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catéchol-borane.

La composition tinctoriale utile dans l'invention contient en général une quantité de colorant fluorescent de formule (I) ou (II) comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises

entre 1 et 40 % en poids environ par rapport au poids total de la composition tinctoriale, et encore plus préférentiellement entre 5 et 30 % en poids environ.

Selon une variante, l'invention contient un agent réducteur capable de réduire les liaisons disulfures de la kératine. Cet agent réducteur est tel que défini précédemment.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule ($\gamma$) suivante :

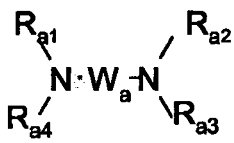

($\gamma$)

dans laquelle $W_a$ est un reste propylène éventuellement substitué par un groupement hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Selon un mode de réalisation particulier dans le procédé de l'invention un agent réducteur peut être appliqué en pré-traitement avant l'application de la composition contenant au moins un colorant fluorescent de formule (I) ou (II).

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Ce prétraitement peut être de courte durée, notamment de 0,1 seconde à 30 minutes, de préférence de 1 minute à 15 minutes avec un agent réducteur tel que cité précédemment.

Selon un autre procédé, la composition comprenant au moins un colorant fluorescent de formule (I) ou (II) contient également au moins un agent réducteur tel que défini précédemment. Cette composition est alors appliquée aux cheveux.

Lorsque le colorant fluorescent thiol de formule (II) comprend un groupement Y protecteur de la fonction thiol, le procédé de l'invention peut être précédé d'une étape de déprotection visant à restituer *in-situ* la fonction SH.

A titre d'exemple il est possible de déprotéger la fonction S-Y avec Y groupement protecteur en ajustant le pH comme suit :

| Y : groupe protecteur | déprotection |
|---|---|
| | |
| al kylcarbonyle, | pH>9 |
| arylcarbonyle, | pH>9 |
| alcoxycarbonyle, | pH>9 |
| aryloxycarbonyle, | pH>9 |
| arylalcoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| hétéroaryle bicyclique à 8 à 11 chaînons tels que benzoimidazolium, ou benzoxazolium | pH>9 |

L'étape de déprotection peut également être réalisée au cour d'une étape de prétraitement des cheveux comme par exemple, le prétraitement réducteur des cheveux.

Selon une variante, l'agent réducteur est ajouté à la composition tinctoriale contenant au moins un colorant fluorescent de formule (I) ou (II) au moment de l'emploi.

Dans le procédé de coloration une autre variante est d'appliquer le colorant fluorescent de formule (I) ou (II) en même temps que le réducteur.

Selon un procédé de l'invention, la composition comprenant au moins un colorant fluorescent de formule (I) ou (II) contient également au moins un agent réducteur tel que défini précédemment. Cette composition est alors appliquée aux cheveux.

Selon une autre variante, l'agent réducteur est appliqué en post-traitement, après l'application de la composition contenant au moins un colorant fluorescent de formule (I) ou (II). La durée du post traitement avec l'agent réducteur peut être courte, par exemple de 0,1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent de formule (I) ou (II) peut être appliqué directement aux cheveux sans réducteurs, exempt de pré ou post-traitement réducteurs.

Un traitement avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant au moins un colorant fluorescent de formule (I) ou (II).

L'application de la composition tinctoriale selon l'invention est généralement effectuée à température ambiante. Elle peut cependant être réalisée à des températures variant de 20 à 180°C.

L'invention a aussi pour objet un dispositif à plusieurs compartiments ou "kit" de teinture dans lequel un premier compartiment renferme une composition tinctoriale comprenant au moins un colorant fluorescent de formule (I) ou (II) et un deuxième compartiment renferme un agent réducteur capable de réduire les fonctions disulfures des matières kératiniques.

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent de formule (I) ou (II) ; un deuxième compartiment contient un agent réducteur capable de réduire la liaison disulfure des matières kératiniques ; un troisième compartiment contient un agent oxydant.

Alternativement, le dispositif de teinture contient un premier compartiment renfermant une composition tinctoriale qui comprend au moins un colorant fluorescent thiol protégé de formule (II) et un deuxième compartiment renfermant

un agent capable de déprotéger le thiol protégé pour libérer le thiol.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents thiols des exemples ci-après ont été entièrement caractérisés par les méthodes spectroscopiques et spectrométriques classiques.

EXEMPLES

EXEMPLES DE SYNTHESE

Exemple 1 : Synthèse du tétrabromure de 1,1'-{disulfanediylbis[éthane-2,1-diyl-(diméthylammonio)butane-4,1-diyl]}bis(2-{(E)-2-[4-(diméthylamino)phényl]-vinyl]pyridinium) [1]

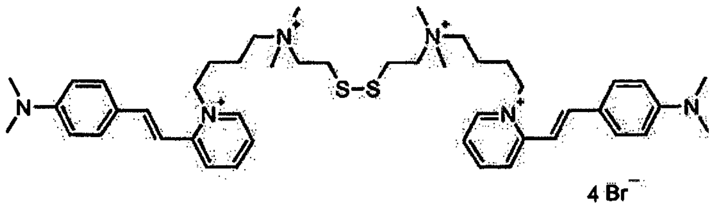

**[1]**

**Schéma de synthèse**

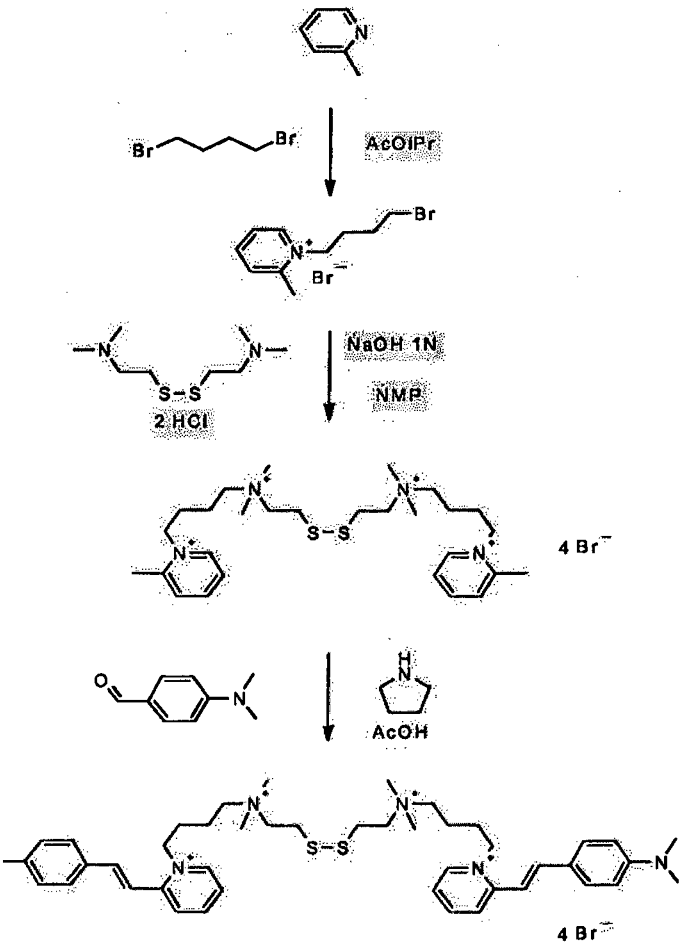


**[1]**

Mode opératoire

Etape 1 : Synthèse du bromure de 1-(4-bromobutyl)-2-méthylpyridinium

12,5 mL de 2-picoline dilués dans 50 mL d'acétate d'isopropyle (AcOiPr) sont ajoutés goutte à goutte à 106 mL de 1,4-dibromobutane et 100 mL d'AcOiPr. Le milieu réactionnel est chauffé à reflux pendant 12 h. 200 mL d'acétate d'éthyle (AcOEt) sont ajoutés au mélange réactionnel préalablement refroidi à température ambiante, le précipité obtenu est filtré puis séché. La poudre obtenue est solubilisée par 100 mL d'eau et extraite par du butanol. La phase butanol est concentrée, puis 100 mL d'AcOEt sont ajoutés au résidu, le précipité obtenu est filtré, puis séché. 20,9 g de poudre marron sont recueillis. Les analyses indiquent que le produit est conforme.

Etape 2 : Synthèse du tétrabromure de 1,1'-{disulfanediylbis[éthane-2,1-diyl(diméthylammonio)butane-4,1-diyl]}bis (2-méthylpyridinium)

A 11,16 g de dichlorhydrate de tétraméthyle cystamine solubilisés dans 50 mL d'eau sont ajoutés 80 mL de soude 1 N (NaOH). Le mélange est concentré à sec. Le résidu est mis en suspension dans 150 mL de dichlorométhane. Le précipité obtenu est filtré. Le filtrat est concentré à sec. L'huile obtenue est concentrée après ajout de 3 fois 50 mL de toluène. 8,3 g de tétraméthyle cystamine sont recueillis. 3,51 g de tétraméthyle cystamine et 10,9 g de bromure de 1-(4-bromobutyl)-2-méthylpyridinium sont mélangés dans 50 mL de N-méthylpyrrolidinone (NMP) sous agitation à 80 °C pendant 48 h. Le chauffage est arrêté; la gomme obtenue est isolée et le surnageant est éliminé. La gomme est solubilisée dans 150 mL d'isopropanol (iPrOH) sous agitation à 80 °C. Après refroidissement, le précipité obtenu est filtré, lavé par 3 fois 50 mL d'iPrOH puis séché sous vide sous $P_2O_5$. 9,6 g de poudre blanche sont recueillis. Les analyses indiquent que le produit est conforme.

Etape 3 : Synthèse du tétrabromure de 1,1'-{disulfanediylbis[éthane-2,1-diyl-(diméthylammonio)butane-4,1-diyl]}bis(2-{(E)2-[4-(diméthylamino)phényl]-vinyl}pyridinium) [1]

0,36 g de 4-diméthylamino-benzaldéhyde en solution dans 2 mL d'isopropanol et 250 µL de pyrrolidine sont agités pendant 10 min à température ambiante, puis pendant 20 min après addition de 143 µL d'acide acétique. 1 g de tétrabromure de 1,1'-{disulfanediylbis[éthane-2,1-diyl(diméthylammonio)butane-4,1-diyl]}bis(2-méthylpyridinium) en suspension dans 2 mL d'isopropanol sont additionnés au milieu réactionnel, maintenu ensuite sous agitation à température ambiante pendant 48h. 50 mL d'acétate d'éthyle sont ajoutés au mélange, le précipité obtenu est filtré, lavé avec 150 mL d'acétate d'éthyle, puis séché. 1,08 g de poudre orange sont recueillis. Les analyses indiquent que le produit est conforme au composé [1]. RMN $^1H$ (400 MHz, MeOH-$d_4$) 2.08 (m, 8 H), 3.04 (s, 12 H), 3.23 (s, 12 H), 3.44 (m, 4H), 3.62 (m, 4 H), 3.77 (m, 4 H), 4.82 (m, 4 H), 6.72 (d, 4 H), 7.23 (d, 2 H), 7.61 (t, 2 H), 7.76 (d, 4 H), 7.79 (d, 2 H), 8.23 (t, 2 H), 8.30 (d, 2 H), 8.76 (d, 2H).

Exemple 2 : Synthèse du tétrabromure de 1,1'-{disulfanediylbis[éthane-2,1-diyl(diméthylammonio)butane-4,1-diyl]}bis {2-[(E)-2-(3,4,5-triméthoxyphényl)-vinyl]pyridinium) [2]

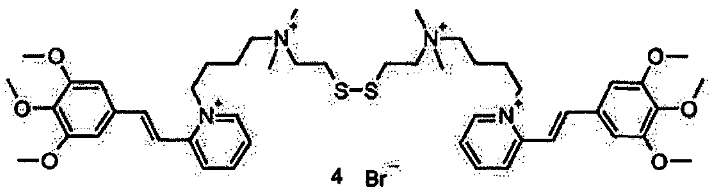

[2]

**Schéma de synthèse**

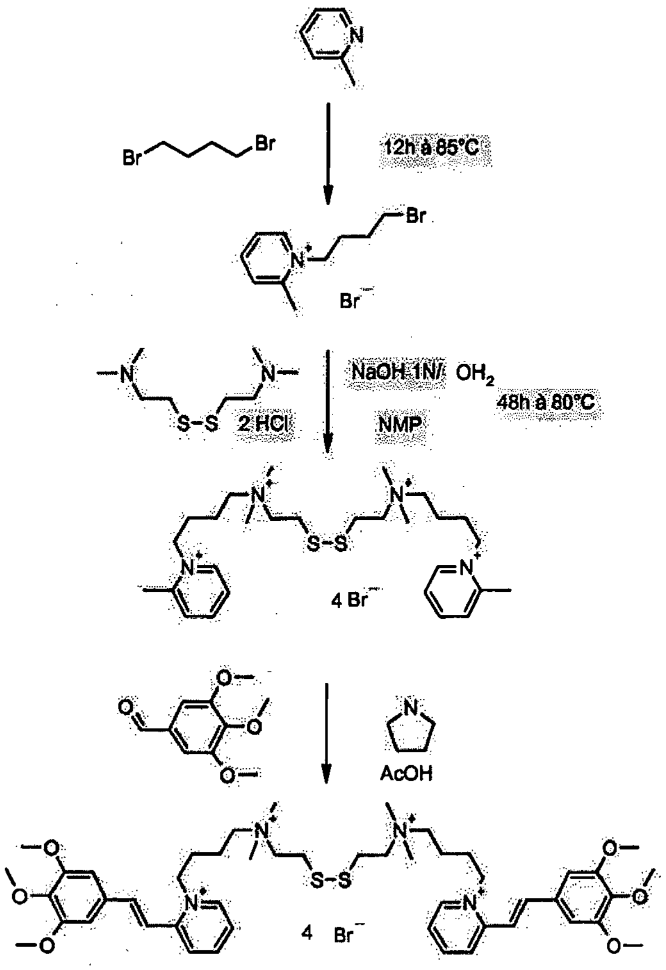


[2]

Mode opératoire

Etape 1 : Synthèse du bromure de 1-(4-bromobutyl)-2-méthylpyridinium

12,5 mL de 2-picoline dilués dans 50 mL d'acétate d'isopropyle (AcOiPr) sont ajoutés goutte à goutte à 106 mL de 1,4-dibromobutane et 100 mL d'AcOiPr. Le milieu réactionnel est chauffé à reflux pendant 12 h. 200 mL d'AcOEt sont ajoutés au mélange réactionnel préalablement refroidi à température ambiante, le précipité obtenu est filtré puis séché. La poudre obtenue est solubilisée par 100 mL d'eau et extraite par du butanol. La phase butanol est concentrée, puis 100 mL d'AcOEt sont ajoutés au résidu, le précipité obtenu est filtré, puis séché. 20,9 g de poudre marron sont recueillis. Les analyses indiquent que le produit est conforme et pur.

Etape 2 : Synthèse du tétrabromure de 1,1'-{disulfanediylbis[éthane-2,1-diyl(diméthylammonio)butane-4,1-diyl]}bis (2-méthylpyridinium)

A 11,16 g de dichlorhydrate de tétraméthyle cystamine solubilisés dans 50 mL d'eau sont ajoutés 80 mL de soude 1 N (NaOH). Le mélange est concentré à sec. Le résidu est mis en suspension dans 150 mL de dichlorométhane. Le précipité obtenu est filtré. Le filtrat est concentré à sec. L'huile obtenue est concentrée après ajout de 3 fois 50 mL de toluène. 8,3 g de tétraméthyle cystamine sont recueillis.
3,51 g de tétraméthyle cystamine et 10,9 g de bromure de 1-(4-bromobutyl)-2-méthylpyridinium sont mélangés dans 50 mL de N-méthylpyrrolidinone (NMP) sous agitation à 80 °C pendant 48h. Le chauffage est arrêté ; la gomme obtenue est isolée et le surnageant est éliminé. La gomme est solubilisée dans 150 mL d'iPrOH sous agitation à 80 °C. Après refroidissement, le précipité obtenu est filtré, lavé par 3 fois 50 mL d'iPrOH puis séché sous vide sous $P_2O_5$. 9,6 g de poudre blanche sont recueillis. Les analyses indiquent que le produit est conforme.

Etape 3 : Synthèse du tétrabromure de 1,1'-{disulfanediylbis[éthane-2,1-diyl(diméthylammonio)butane-4,1-diyl]}bis{2-[(E)-2-(3,4,5-triméthoxyphényl)vinyl]pyridinium}[2]

0,47 g de 3,4,5-triméthoxy-benzaldéhyde, 2 mL d'iPrOH, 205 μL de pyrrolidine sont agités à température ambiante pendant 10 minutes. 143 μL d'acide acétique sont ajoutés au milieu maintenu sous agitation pendant 20 minutes. 1 g de Tétrabromure de 1,1'-{disulfanediylbis[éthane-2,1diyl(diméthylammonio)butane-4,1-diyl]}bis(2-méthylpyridinium) solubilisé dans 2 mL d'iPrOH est introduit au milieu réactionnel. Après trois jours d'agitation à température ambiante, 50 mL d'AcOEt sont ajoutés au mélange. Le précipité obtenu est filtré, lavé avec 50 mL d'AcOEt puis séché. 1,3 g de poudre sont recueillis. Les analyses indiquent que le produit est conforme. LCMS : pic de masse m/z = 217, correspondant au tétracation. $\lambda_{max}$ 414 nm.

<u>Exemple 3</u> : synthèse du tétrabromure de 1,1'-{disulfanediylbis[éthane-2,1-diyl(dimethylammonio)butane-4,1-diyl]}bis[2-((E)-2-{4-[bis(2-hydroxyéthyl)amino]phényl}vinyl)pyridinium] [3]

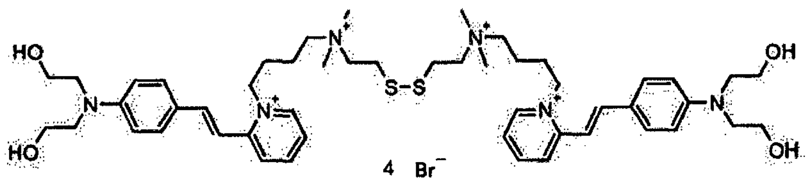

[3]

## Schéma de synthèse

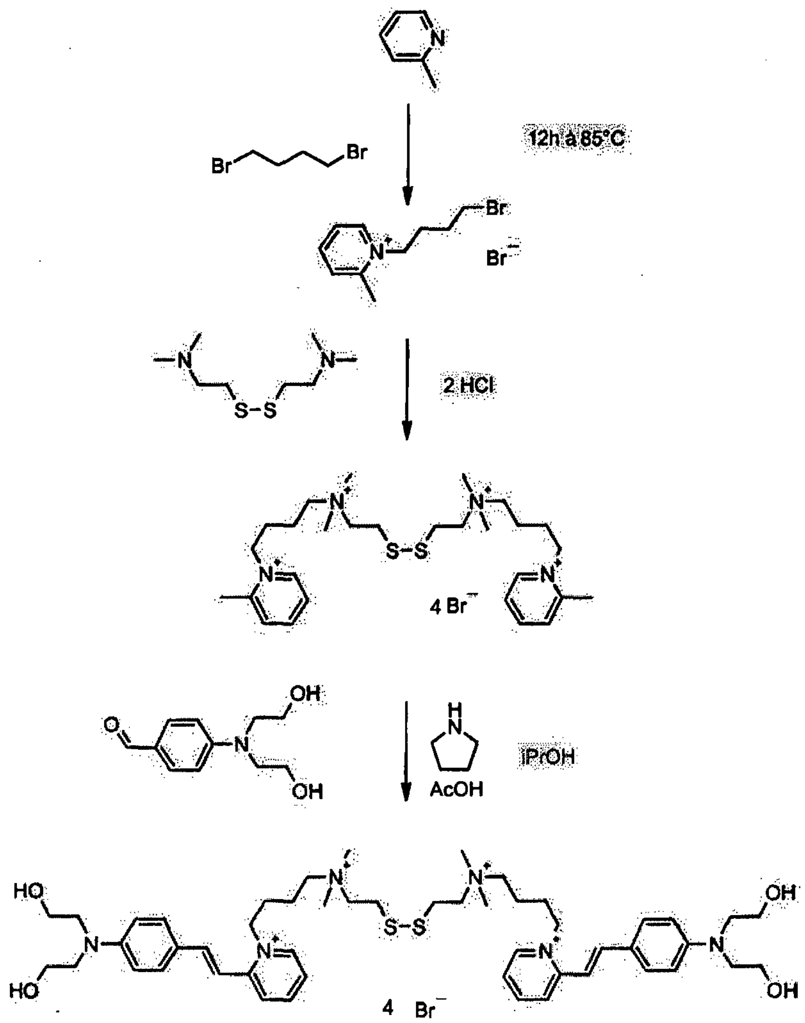


[3]

Mode opératoire

Etape 1 : Synthèse du bromure de 1-(4-bromobutyl)-2-méthylpyridinium

12,5 mL de 2-picoline dilués dans 50 mL d'acétate d'isopropyle (AcOiPr) sont ajoutés goutte à goutte à 106 mL de 1,4-dibromobutane et 100 mL d'acétate d'AcOiPr. Le milieu réactionnel est chauffé à reflux pendant 12 h. 200 mL d'AcOEt sont ajoutés au mélange réactionnel préalablement refroidi à température ambiante, le précipité obtenu est filtré puis séché. La poudre obtenue est solubilisée par 100 mL d'eau et extraite par du butanol. La phase butanol est concentrée, puis 100 mL d'AcOEt sont ajoutés au résidu, le précipité obtenu est filtré, puis séché. 20,9 g de poudre marron sont recueillis. Les analyses indiquent que le produit est conforme et pur.

Etape 2 : Synthèse du tétrabromure de 1,1'-{disulfanediylbis[éthane-2,1-diyl-(diméthylammonio)butane-4,1-diyl]}bis (2-méthylpyridinium)

A 11,16 g de dichlorhydrate de tétraméthyle cystamine solubilisés dans 50 mL d'eau sont ajoutés 80 mL de soude 1 N (NaOH). Le mélange est concentré à sec. Le résidu est mis en suspension dans 150 mL de dichlorométhane. Le précipité obtenu est filtré. Le filtrat est concentré à sec. L'huile obtenue est concentrée après ajout de 3 fois 50 mL de toluène. 8,3 g de tétraméthyle cystamine sont recueillis. 3,51 g de tétraméthyle cystamine dilués et 10,9 g de bromure de 1-(4-bromobutyl)-2-méthylpyridinium sont mélangés dans 50 mL de NMP sous agitation à 80 °C pendant 48 h. Le chauffage est arrêté ; la gomme obtenue est isolée et le surnageant est éliminé. La gomme est solubilisée dans 150

mL d'iPrOH sous agitation à 80 °C. Après refroidissement, le précipité obtenu est filtré, lavé par 3 fois 50 mL d'iPrOH puis séché sous vide sous $P_2O_5$. 9,6 g de poudre blanche sont recueillis. Les analyses indiquent que le produit est conforme.

Etape 3 : Synthèse du tétrabromure de 1,1'-{disulfanediylbis[éthane-2,1-diyl(dimethylammonio)butane-4,1-diyl]}bis [2-((E)-2-{4-[bis(2-hydroxyéthyl)-amino]phényl)vinyl)pyridinium] [3]

420 mg de 4-[bis(2-hydroxyéthyl)amino]benzaldéhyde et 826 mg de tétrabromure de 1,1'-{disulfanediylbis [éthane-2,1diyl(diméthylammonio)butane-4,1-diyl]}bis(2-méthylpyridinium) sont mis en suspension dans 5 mL d'iPrOH et le mélange est proté à 70 °C. 134 µL de pyrrolidine sont ajoutés et l'agitation est maintenue à agités à 70 °C pendant 3 h 30 après refroidissement. Une huile décante après refroidissement. Elle est séparée du surnageant et lavée par 2 fois 5 mL d'isopropanol à chaud, reprise dans 5 mL de méthanol et versée sur 200 mL de tertiobutylméthyl éther. Le précipité est filtré et séché sous vide. 489 mg de poudre noire sont recueillis. Les analyses indiquent que le produit est conforme. LCMS : pic de masse m/z = 222, correspondant au tétracation). Lambda max 450 nm

<u>Exemple 4</u> : synthèse du dibromure de 2-{(E)-2-[4-(diméthylamino)phényl]vinyl}-1-{4-[diméthyl(2-sulfanyléthyl)ammonio]butyl}pyridinium [4]

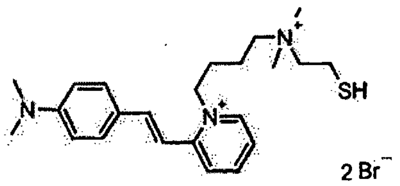

**[4]**

**Schéma de synthèse**

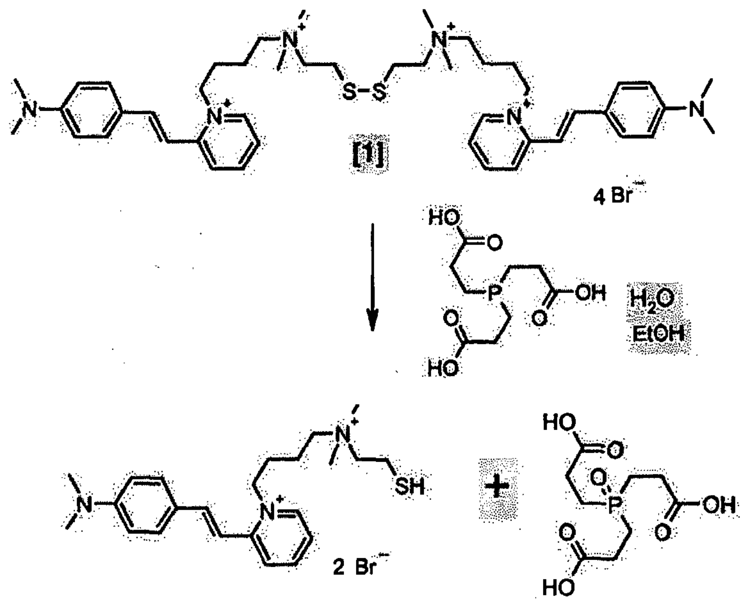


Synthèse du sel de 2-{(E)-2-[4-(diméthylamino)phényl]vinyl}-1-{4-[diméthyl(2-sulfanyléthyl)ammonio]butyl}pyridinium [4]

110 mg du composé [1] sont dissous dans 10 mL de mélange eau/éthanol (v/v 1/1) ; 60 mg (2 éq.) d'hydrate de chlorhydrate d'acide 3-[bis(2-carboxy-éthyl)phosphino]- propanoïque en solution dans 1 mL d'eau et 21 mg (4 éq) de bicarbonate de sodium en solution dans 1 mL d'eau sont ajoutés au mélange. Après 30 minutes d'agitation à 40°C sous atmosphère inerte, les analyses indiquent que le mélange contient très majoritairement le produit attendu [4].

Analyse LC-MS : LC-DAD (400-700 nm)
Colonne : Waters XTerra MS C18 5μm (4.6 x 50) mm
Phase Mobile : A : Eau + acide formique 0.1% / B : acétonitrile
Gradient linéaire : T (min) A%/B% : 0 min 95/5 ; 8 min 0/100
Débit: 1mL/min.
Détection : UV Barrette de diodes λ=400-700nm

Temps de rétention t = 3.2 min.
Pureté relative >95%
MS (ESI+) m/z=193 correspond au pic de masse du dication du produit attendu [4] HRMS (ESI+) m/z = 192,6270 ($^{12}C_{23}H_{35}{}^{14}N_3{}^{32}S_1$ Δm/m : - 0.09 ppm)

## EXEMPLE DE COLORATION

### Exemple 1 - procédé de coloration avec le composé [1]

Préparation d'une composition A

| | |
|---|---:|
| Colorant disulfure [1] | $10^{-3}$ mol% |
| Alcool Benzylique | 4 g |
| Polyéthylèneglycol 60E | 6 g |
| Hydroxyethylcellulose | 0,7 g |
| Alkylpolyglucoside en solution aqueuse à 65%MA | 4.5 g |
| Eau déminéralisée | qsp 100g |

Préparation d'une composition B

| | |
|---|---:|
| Acide thioglycolique | 1 M |
| Hydroxyde de Sodium | qsp pH 8,5 |
| Eau déminéralisée | qsp 100g |

Au moment de l'emploi, on mélange les compositions A (9 mL) et B (1 mL), puis on applique le mélange obtenu sur une mèche de 1 g de cheveux foncés (hauteur de ton 4) pendant 30 minutes à température ambiante(les mèches sont retournées et réimprégnées après 15 minutes).

Après rinçage à l'eau courante et séchage, on observe un éclaircissement des cheveux ainsi traités : la mèche de hauteur de ton 4 est devenue visuellement plus claire que des mèches témoins non traitées.

Exemple 2 - procédé de coloration avec le composé [4]

Procédé de coloration

10 mL de solution fraîche du composé [4] de l'exemple de synthèse sont appliqués sur une mèche de 1 g cheveux de hauteur de ton 4 disposées au fond d'un même bol pendant 30 minutes à température ambiante (les mèches sont retournées et réimprégnées après 15 minutes).

Les mèches sont ensuite rincées à l'eau courante et séchées.

Après coloration la mèche de hauteur de ton 4 est devenue visuellement plus claire que des mèches témoins non traitées.

Rémanence vis-à-vis des shampooings successifs :

Les mèches ainsi traitées par selon l'exemple de coloration [1] ou [4] sont divisées en deux, une moitié est soumise à 5 shampooings successifs selon un cycle qui comprend le mouillage des mèches à l'eau, le lavage avec un shampooing classique, un rinçage à l'eau suivi d'un séchage.

*Observations visuelles*

Lors des shampooings, il n'y a pas de dégorgement visible, la mousse des shampooings et les eaux de rinçage ne sont pas colorées

La couleur observée sur les mèches est conservée et l'effet d'éclaircissement reste visible sur les cheveux ainsi traités

*Résultats dans le système L*a*b* :*

La couleur des mèches avant et après les 5 lavages a été évaluée dans le système L*a*b* au moyen d'un spectrophotomètre CM 2600D MINOLTA ®, (Illuminant D65).

Dans ce système L* a* b*, L* représente la luminosité, a* indique l'axe de couleur vert/rouge et b* l'axe de couleur bleu/jaune. Plus la valeur de L est élevée, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.

La variation de la coloration entre les mèches de cheveux HT4, colorées et lavées est mesurée par (ΔE) selon l'équation suivante :

$$\Delta E = \sqrt{(L^* - L_o^*)^2 + (a^* - a_o^*)^2 + (b^* - b_o^*)^2}$$

Dans cette équation, L*, a* et b* représentent les valeurs mesurées avant coloration et $L_0$*, $a_0$* et $b_0$* représentent les valeurs mesurées avant coloration (ou shampooing).

Plus la valeur de delta E est importante, plus la différence de couleur entre les mèches HT4 et les mèches colorées est importante.

| Traitement avec le colorant fluorescent sur les mèches de HT4 | Δ E |
|---|---|
| | |
| Après application du composé 1 selon l'invention | 6,65 |
| Après application du composé 1 selon l'invention et après 5 shampooings successifs | 7,40 |

Les résultats du tableau ci-dessus montrent que la coloration évolue très peu après 5 shampooings. Ainsi la coloration et l'effet éclaircissant sur les cheveux restent quasiment inchangés ce qui montre une très bonne résistance aux shampooings des colorants de l'invention.

*Résultats de Réflectance :*

Les performances d'éclaircissement des compositions conformes à l'invention et leur rémanence vis-à-vis de shampooings successifs ont été exprimées en fonction de la réflectance des cheveux. Ces réflectances sont comparées à la réflectance d'une mèche de cheveux non traitées de hauteur de ton HT4 .

La réflectance est mesurée au moyen d'un appareil spectrophotocolorimètre KONIKA-MINOLTA®, CM 2600d et après irradiation des cheveux avec une lumière visible dans la gamme de longueurs d'onde allant de 400 à 700 nanomètres.

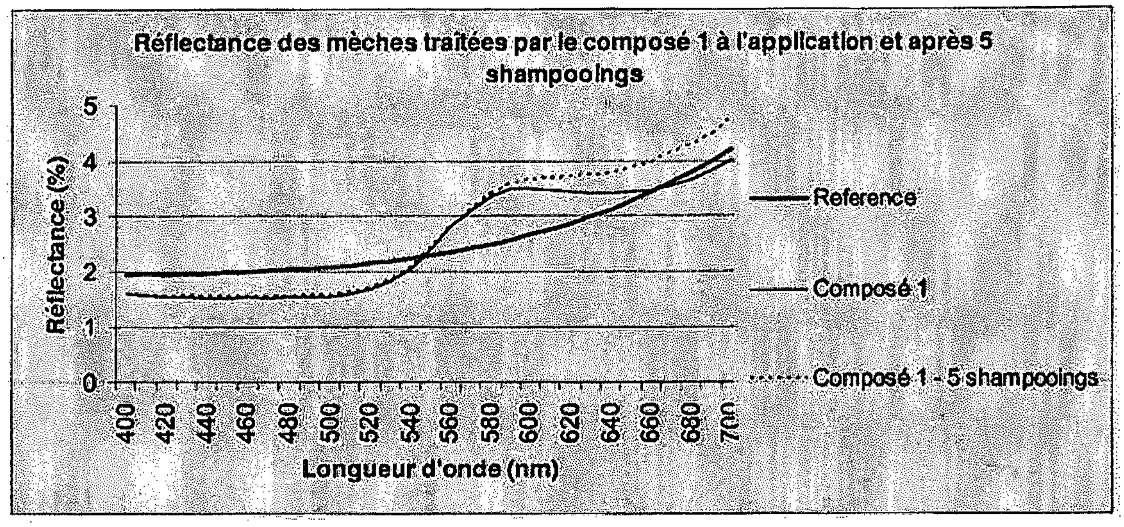


On constate tout d'abord que la réflectance d'une mèche de cheveux traitée avec une composition selon l'invention est supérieure à celle des cheveux non traités. Les mèches traitées apparaissent donc plus claires.

De plus, les résultats montrent que la réflectance des mèches de cheveux de hauteur de ton 4 traitées avec les compositions de l'invention évoluent très peu après 5 shampooings. Ainsi la coloration et l'effet éclaircissant sur les cheveux restent quasiment inchangés ce qui montre une très bonne résistance aux shampooings des colorants de l'invention.

## Revendications

1. Colorant fluorescent de formule (I) ou (II) suivante :

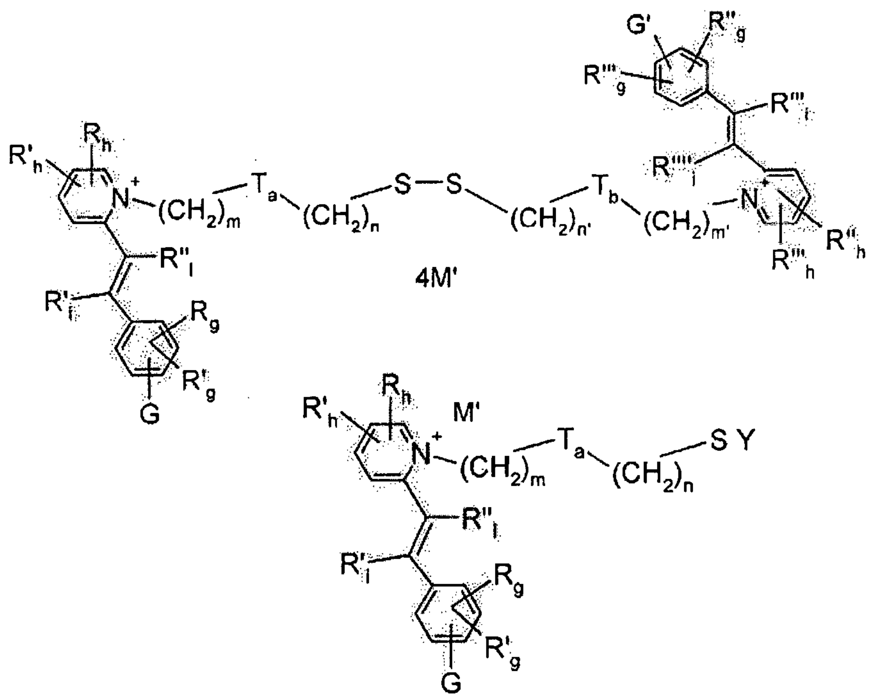

(I)

(II)

ses sels d'acide organique ou minéral, isomères optiques, isomères géométriques, et les solvates tels que hydrates ; formules (I) et (II) dans laquelle :

➢ G et G', identiques ou différents, représentent un groupement $-NR_cR_d$ ou $C_1$-$C_6$ alcoxy éventuellement

substitué ;

➢ $R_c$ et $R_d$ identiques ou différents, représentent un atome d'hydrogène, un groupement aryl($C_1$-$C_4$)alkyle, ou un groupement ($C_1$-$C_6$)alkyle éventuellement substitué ;

➢ $R_g$, $R'_g$, $R''_g$, $R'''_g$, $R_h$, $R'_h$, $R''_h$ et $R'''_h$, identiques ou différents, représentent un atome d'hydrogène, d'halogène, un groupement amino, (di)($C_1$-$C_4$)alkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy ($C_1$-$C_4$)alcoxycarbonyle, ($C_1$-$C_4$)alkylcarbonylamino, acylamino, carbamoyle, ($C_1$-$C_4$)alkylsulfonylamino, un radical amino-sulfonyle, ou un radical ($C_1$-$C_{16}$) alkyle éventuellement substitué par un groupement choisi parmi ($C_1$-$C_{12}$)alcoxy, hydroxy, cyano, carboxy, amino, (di)($C_1$-$C_4$)alkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

➢ $R'_i$, $R''_i$, $R'''_i$ et $R''''_i$, identiques ou différents, représentent un atome d'hydrogène, ou un groupement ($C_1$-$C_4$) alkyle ;

➢ $T_a$ et $T_b$, identiques ou différents, représentent :

- soit un radical ammonium $-N^+(R)(R°)-$, avec R et R°, identiques ou différents, représentant un atome d'hydrogène, un radical alkyle en $C_1$-$C_4$, hydroxyalkyle en $C_1$-$C_4$ ou un aryl($C_1$-$C_4$)alkyle ;
- soit un radical hétérocycloalkyle ou hétéroaryle, cationique préférentiellement monocycliques, contenant notamment deux hétéroatomes, particulièrement deux atomes d'azote, et comportant notamment de 5 à 7 chaînons tel que l'imidazolium, pyridinium ou pyrolidinium éventuellement substitué par un groupement ($C_1$-$C_4$) alkyle tel que méthyle ;

➢ m, m', n et n', identiques ou différents, représentent un entier compris inclusivement entre 0 et 6 avec m+n, m'+n' représentent un entier compris inclusivement entre 1 et 10;

➢ M', identiques ou différents, représentant un contre-ion anionique ; et

➢ Y représente : i) un atome d'hydrogène ; ii) un métal alcalin ; iii) un métal alcalino-terreux ; iv) un groupement ammonium : $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe ($C_1$-$C_4$)alkyle ; ou v) un groupement protecteur de fonction thiol ;

étant entendu que lorsque le composé de formule (I) ou (II) contient d'autres parties cationiques, il se trouve associé à un ou plusieurs contre-ions anioniques permettant d'atteindre l'électroneutralité de la formule (I) ou (II).

**2.** Colorant fluorescent de formule (II) selon la revendication précédente dans lequel Y représente un atome d'hydrogène, ou un métal alcalin.

**3.** Colorant fluorescent de formule (II) selon la revendications 1 dans lequel Y représente un groupement protecteur.

**4.** Colorant fluorescent de formule (II) selon la revendication précédente dans lequel Y représente un groupement protecteur choisi parmi les radicaux suivants :

➢ ($C_1$-$C_4$)alkylcarbonyle ;
➢ ($C_1$-$C_4$)alkylthiocarbonyle ;
➢ ($C_1$-$C_4$)alcoxycarbonyle ;
➢ ($C_1$-$C_4$)alcoxyhiocarbonyle ;
➢ ($C_1$-$C_4$)alkylthio-thiocarbonyle ;
➢ (di) ($C_1$-$C_4$) (alkyl)aminocarbonyle ;
➢ (di) ($C_1$-$C_4$) (alkyl)aminothiocarbonyle ;
➢ arylcarbonyle ;
➢ aryloxycarbonyle ;
➢ aryl($C_1$-$C_4$)alcoxycarbonyle ;
➢ (di) ($C_1$-$C_4$) (alkyl)aminocarbonyle ;
➢ ($C_1$-$C_4$)(alkyl)arylaminocarbonyle ;
➢ carboxy;
➢ $SO_3^-$ ; $M^+$ avec $M^+$ représentant un métal alcalin ou alors $M^+$ et M' de la formule (II) sont absents ;
➢ aryle éventuellement substitué,
➢ hétéroaryle éventuellement substitué;
➢ hétérocycloalkyle éventuellement substitué, éventuellement cationique ;

➢ isothiouronium $-C(NR'^cR'^d)=N^+R'^eR'^f$; $An^-$ avec $R'^c$, $R'^d$, $R'^e$ et $R'^f$, identiques ou différents représentent un atome d'hydrogène ou un groupement alkyle ; et Anreprésente un contre-ion anionique ;
➢ isothiourée $-C(NR'^cR'^d)=NR'^e$ avec $R'^c$, $R'^d$, $R'^e$, tels que définis précédemment ;
➢ (di)arylalkyle éventuellement substitué ;
➢ (di)hétéroarylakyle éventuellement substitué ;
➢ $CR'^1R'^2R'^3$ avec $R'^1$, $R'^2$ et $R'^3$ identiques ou différents, représentant un atome d'halogène ou un groupe choisi parmi :

- alkyle ;
- alcoxy ;
- aryle éventuellement substitué ;
- hétéroaryle éventuellement substitué ;

➢ $P(Z^1)R''^1R''^2R''^3$ avec $R''^1$, et $R''^2$ identiques ou différents représentent un groupement hydroxy, alcoxy ou alkyle, $R''^3$ représente un groupement hydroxy ou alcoxy, et $Z^1$ représente un atome d'oxygène ou de soufre ;
➢ cyclique stériquement encombré tel que le groupe adamantyle ; et
➢ alcoxy($C_1$-$C_4$)alkyle éventuellement substitué.

**5.** Colorant fluorescent de formule (II) selon l'une quelconque des revendications précédentes dans lequel Y représente un métal alcalin ou un groupement protecteur choisi parmi :

➢ ($C_1$-$C_4$)alkylcarbonyle
➢ arylcarbonyle ;
➢ ($C_1$-$C_4$)alcoxycarbonyle ;
➢ aryloxycarbonyle ;
➢ aryl($C_1$-$C_4$)alcoxycarbonyle ;
➢ (di) ($C_1$-$C_4$) (alkyl)aminocarbonyle ;
➢ ($C_1$-$C_4$)(alkyl)arylaminocarbonyle ;
➢ aryle éventuellement substitué ;
➢ hétéroaryle monocyclique à 5 ou 6 chaînons ;
➢ hétéroaryle bicyclique cationique à 8 à 11 chaînons ;
hétérocycle cationique de formule suivante :

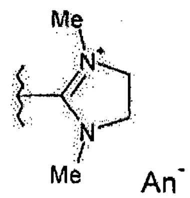

➢ isothiouronium $-C(NH_2)=N^+H_2$; $An^-$;
➢ isothiourée $-C(NH_2)=NH$ ; et
➢ $SO_3^-$ ; $M^+$ avec $M^+$ représentant un métal alcalin ou alors $M^+$ et M' de la formule (II) sont absents.

**6.** Colorant fluorescent de formule (I) ou (II) selon l'une quelconque des revendications précédentes, de formule suivante :

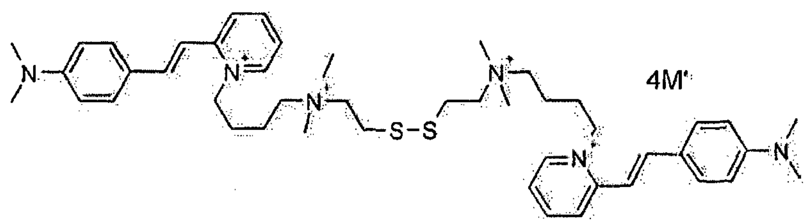

**1**

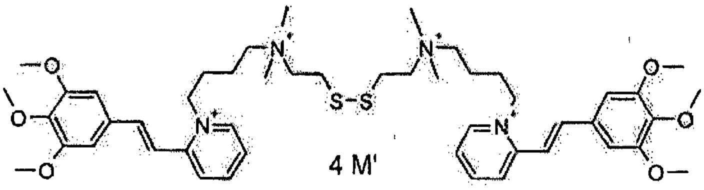

4 M'

**2**

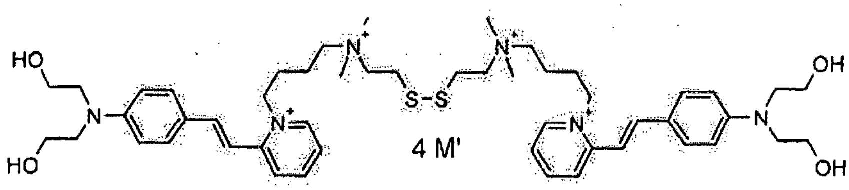

4 M'

**3**

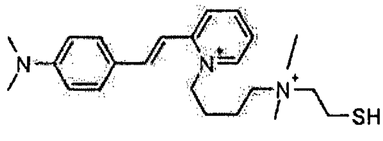

2M'

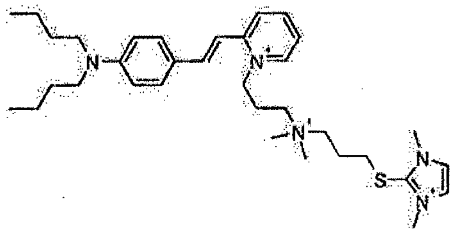

3M'

**4**

**5**

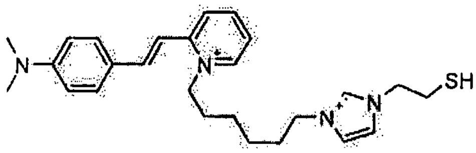

2M'

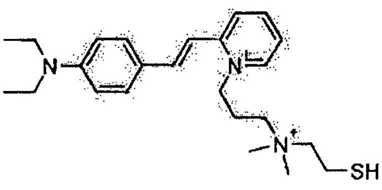

2M'

**6**

**7**

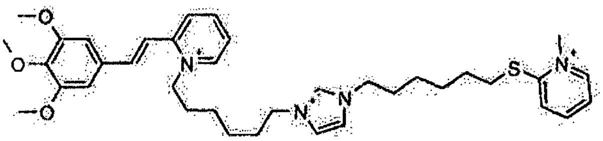

3M'

2M'

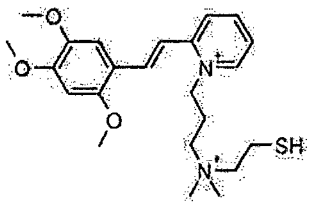

**8**

**9**

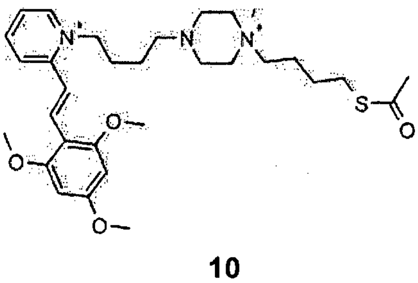

2M'

**10**

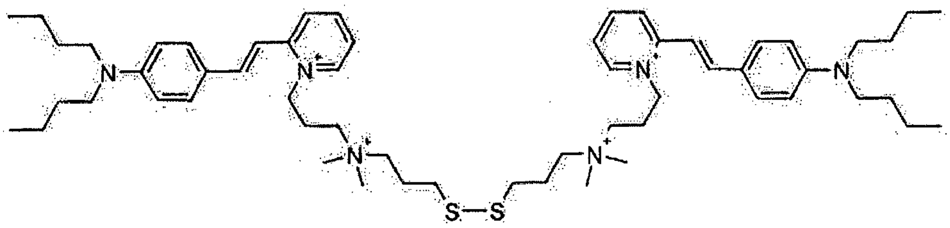

4M'

**11**

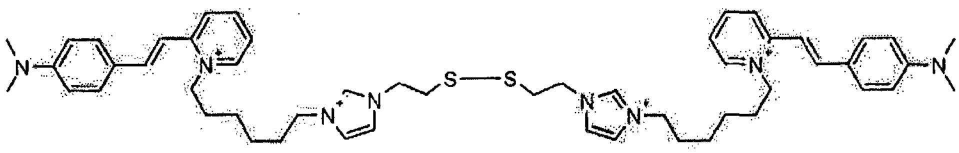

4M'

**12**

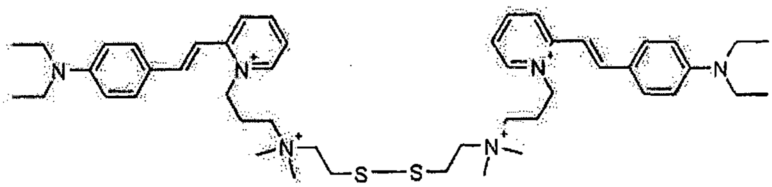

4M'

**13**

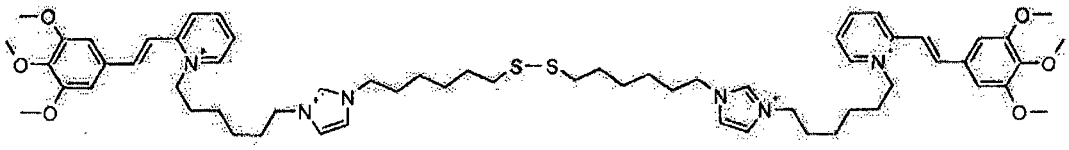

4M'

**14**

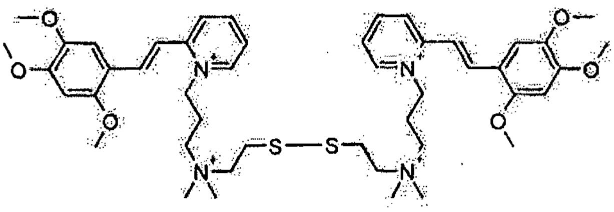

4M'

**15**

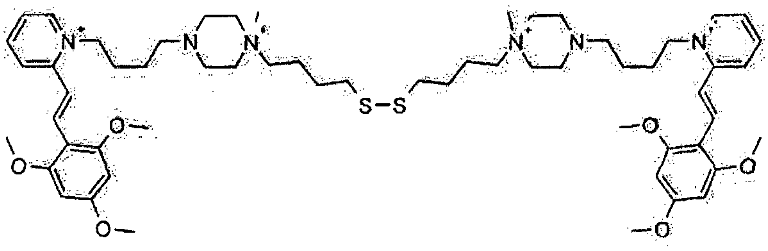

4M'

**16**

Avec M', identiques ou différents, représentant un contre-ion anionique.

7. Composition tinctoriale comprenant dans un milieu cosmétique approprié, un colorant fluorescent de formule (I) ou (II) tel que défini dans une quelconque des revendications 1 à 6.

8. Composition tinctoriale comprenant dans un milieu cosmétique approprié, au moins un colorant fluorescent de formule (I) ou (II) tel que défini dans une quelconque des revendications 1 à 6 et au moins un agent réducteur.

9. Composition selon les revendications 7 ou 8, dans laquelle le colorant fluorescent de formule (I) ou (II) est présent en quantité comprise entre 0,001 et 50% en poids par rapport au poids total de la composition.

10. Procédé de coloration de matières kératiniques, dans lequel on applique sur les matières, une composition tinctoriale telle que définie dans une quelconque des revendications 7 à 9, comprenant dans un milieu cosmétique approprié, au moins un colorant fluorescent de formule (I) ou (II) tel que défini dans une quelconque des revendications 1 à 6, éventuellement en présence d'un agent réducteur capable de réduire les liaisons disulfures des matières kératiniques.

11. Procédé de coloration de matières kératiniques selon la revendication 10 **caractérisé en ce que** les matières kératiniques sont des fibres kératiniques foncées possédant une hauteur de ton inférieure ou égale à 6.

12. Procédé selon les revendications 10 ou 11, dans lequel l'agent réducteur est appliqué avant ou après l'application du colorant fluorescent thiol.

13. Procédé selon l'une quelconque des revendications 10 à 12 comprenant une étape supplémentaire consistant à appliquer sur les fibres kératiniques un agent oxydant.

14. Dispositif à plusieurs compartiments dans lequel un premier compartiment contient une composition tinctoriale comprenant un colorant fluorescent tel que défini aux revendications 1 à 6 et un deuxième compartiment contient un agent réducteur capable de réduire les liaisons disulfures des matières kératiniques.

15. Utilisation des colorants fluorescents tels que définis aux revendications 1 à 6 pour l'éclaircissement des fibres kératiniques foncées qui possèdent une hauteur de ton inférieure à 6.

**Patentansprüche**

1. Fluoreszenzfarbstoff der folgenden Formel (I) oder (II) :

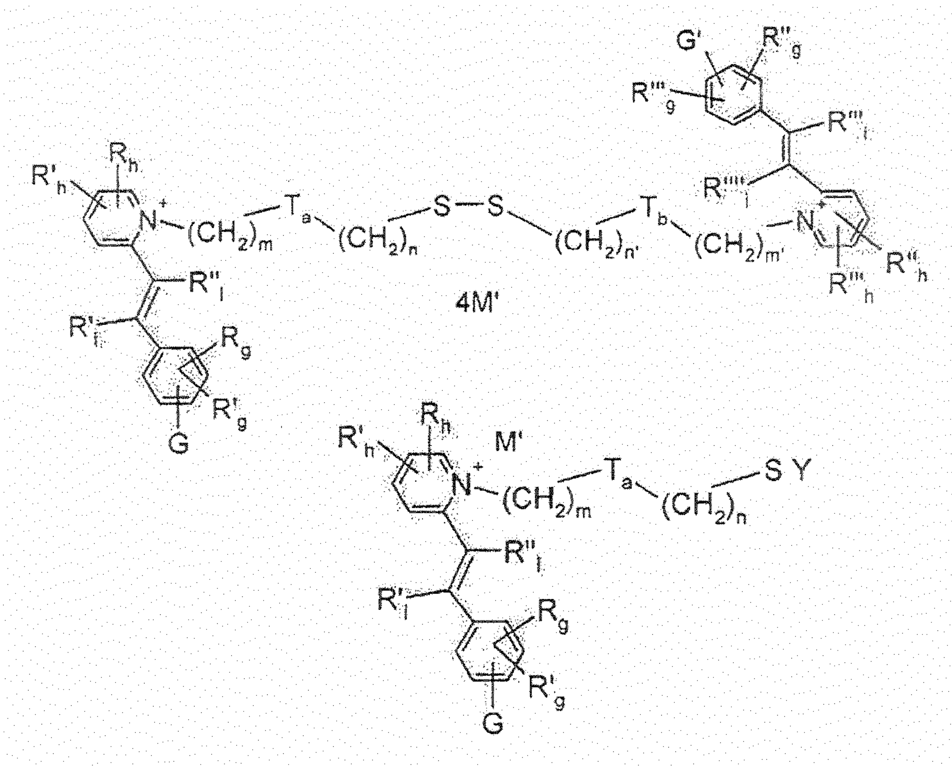

(I)

(II)

Salze davon mit einer organischen oder anorganischen Säure, optische Isomere davon, geometrische Isomere davon und Solvate wie Hydrate;
wobei in den Formeln (I) und (II):

➢ G und G' gleich oder verschieden sind und für eine $-NR_cR_d$-Gruppe oder $C_1$-$C_6$-Alkoxygruppe, die gegebenenfalls substituiert ist, stehen;

➢ $R_c$ und $R_d$ gleich oder verschieden sind und für ein Wasserstoffatom, eine Aryl($C_1$-$C_4$)alkylgruppe oder eine gegebenenfalls substituierte ($C_1$-$C_6$) - Alkylgruppe stehen;

➢ $R_g$, $R'_g$, $R''_g$, $R'''_g$, $R_h$, $R'_h$, $R''_h$ und $R'''_h$ gleich oder verschieden sind und für ein Wasserstoff- oder Halogenatom, eine Amino-, (Di) ($C_1$-$C_4$)alkylamino-, Cyano-, Carboxy-, Hydroxy-, Trifluormethyl-, Acylamino-, $C_1$-$C_4$-Alkoxy-, $C_2$-$C_4$-(Poly) hydroxyalkoxy-, ($C_1$-$C_4$)-Alkylcarbonyloxy-, ($C_1$-$C_4$)Alkoxycarbonyl-, ($C_1$-$C_4$)Alkylcarbonylamino-, Acylamino-, Carbamoyl- oder ($C_1$-$C_4$)Alkylsulfonylamino- gruppe, einen Aminosulfonylrest oder einen ($C_1$-$C_{16}$)Alkylrest, der gegebenenfalls durch eine aus ($C_1$-$C_{12}$) Alkoxy, Hydroxy, Cyano, Carboxy, Amino und (Di) ($C_1$-$C_4$)alkylamino ausgewählte Gruppe substituiert ist, stehen oder die beiden Alkylreste, die an das Stickstoffatom der Aminogruppe gebunden sind, einen 5- bis 7-gliedrigen Heterocyclus bilden, der gegebenenfalls ein weiteres mit dem Stickstoffatom identisches oder davon verschiedenes Heteroatom enthält;

➢ $R'_i$, $R''_i$, $R'''_i$ und $R''''_i$ gleich oder verschieden sind und für ein Wasserstoffatom oder eine ($C_1$-$C_4$)-Alkylgruppe stehen;

➢ $T_a$ und $T_b$ gleich oder verschieden sind und für:

- einen Ammoniumrest $-N^+(R)(R°)-$, wobei R und R° gleich oder verschieden sind und für ein Wasserstoffatom, einen $C_1$-$C_4$-Alkyl- oder $C_1$-$C_4$-Hydroxyalkylrest oder ein Aryl($C_1$-$C_4$)alkyl stehen
- oder einen kationischen Heterocycloalkyl-oder Heteroarylrest, der vorzugsweise monocyclisch ist, insbesondere zwei Heteroatome, speziell zwei Stickstoffatome, enthält und insbesondere 5- bis 7-gliedrig ist, wie Imidazolium, Pyridinium oder Pyrrolidinium, gegebenenfalls substituiert durch eine ($C_1$-$C_4$)Alkylgruppe wie Methyl;

stehen;

➢ m, m', n und n' gleich oder verschieden sind und für eine ganze Zahl zwischen 0 und 6 einschließlich stehen, wobei m+n und m'+n' für eine ganze Zahl zwischen 1 und 10 einschließlich stehen;

➢ die Gruppen M' gleich oder verschieden sind und für ein anionisches Gegenion stehen und

➢ Y für Folgendes steht: i) ein Wasserstoffatom; ii) ein Alkalimetall; iii) ein Erdalkalimetall; iv) eine Ammonium-

gruppe: $N^+R^\alpha R^\beta R^\gamma R^\delta$ oder eine Phosphoniumgruppe: $P^+R^\alpha R^\beta R^\gamma R^\delta$, wobei $R^\alpha$, $R^\beta$, $R^\gamma$ und $R^\delta$ gleich oder verschieden sind und für ein Wasserstoffatom oder eine $(C_1-C_4)$Alkylgruppe stehen; oder v) eine Schutzgruppe für die Thiolfunktion;

mit der Maßgabe, dass die Verbindung der Formel (I) oder (II) dann, wenn sie andere kationische Teile enthält, mit einem oder mehreren anionischen Gegenionen assoziiert ist, so dass die Elektroneutralität der Formel (I) oder (II) gewährleistet ist.

**2.** Fluoreszenzfarbstoff der Formel (II) nach dem vorhergehenden Anspruch, worin Y für ein Wasserstoffatom oder ein Alkalimetall steht.

**3.** Fluoreszenzfarbstoff der Formel (II) nach Anspruch 1, worin Y für eine Schutzgruppe steht.

**4.** Fluoreszenzfarbstoff der Formel (II) nach einem der vorhergehenden Ansprüche, worin Y für eine aus den folgenden Resten ausgewählte Schutzgruppe steht:

- ➢ $(C_1-C_4)$Alkylcarbonyl;
- ➢ $(C_1-C_4)$Alkylthiocarbonyl;
- ➢ $(C_1-C_4)$Alkoxycarbonyl;
- ➢ $(C_1-C_4)$Alkoxythiocarbonyl;
- ➢ $(C_1-C_4)$Alkylthiothiocarbonyl;
- ➢ (Di) $(C_1-C_4)$(alkyl)aminocarbonyl ;
- ➢ (Di) $(C_1-C_4)$(alkyl)aminothiocarbonyl;
- ➢ Arylcarbonyl;
- ➢ Aryloxycarbonyl;
- ➢ Aryl $(C_1-C_4)$alkoxycarbonyl;
- ➢ (Di) $(C_1-C_4)$ (alkyl)aminocarbonyl;
- ➢ $(C_1-C_4)$(Alkyl)arylaminocarbonyl;
- ➢ Carboxy;
- ➢ $SO_3^-$; $M^+$, wobei $M^+$ für ein Alkalimetall steht oder auch $M^+$ und M' der Formel (II) fehlen;
- ➢ gegebenenfalls substituiertem Aryl;
- ➢ gegebenenfalls substituiertem Heteroaryl;
- ➢ gegebenenfalls kationischem, gegebenenfalls substituiertem Heterocycloalkyl;

  - ■ Isothiouronium $-C(NR'^cR'^d)=N^+R'^eR'^f$; $An^-$, wobei $R'^c$, $R'^d$, $R'^e$ und $R'^f$ gleich oder verschieden sind und für ein Wasserstoffatom oder eine Alkylgruppe stehen und $An^-$ für ein anionisches Gegenion steht;

- ➢ Isothioharnstoff $-C(NR'^cR'^d)=NR'^e$, wobei $R'^c$, $R'^d$ und $R'^e$ die oben angegebene Bedeutung besitzen;
- ➢ gegebenenfalls substituiertem (Di)arylalkyl;
- ➢ gegebenenfalls substituiertem (Di)heteroaryl-alkyl;
- ➢ $CR'^1R'^2R'^3$, wobei $R'^1$, $R'^2$ und $R'^3$ gleich oder verschieden sind und für ein Halogenatom oder eine unter:

  - ■ Alkyl;
  - ■ Alkoxy;
  - ■ gegebenenfalls substituiertem Aryl;
  - ■ gegebenenfalls substituiertem Heteroaryl;

  ausgewählte Gruppe stehen;
- ➢ $P(Z^1)R''^1R''^2R''^3$, wobei $R''^1$ und $R''^2$ gleich oder verschieden sind und für eine Hydroxy-, Alkoxy- oder Alkylgruppe stehen, $R''^3$ für eine Hydroxy- oder Alkoxygruppe steht und $Z^1$ für ein Sauerstoff- oder Schwefelatom steht;
- ➢ einer sterisch gehinderten cyclischen Gruppe wie der Adamantylgruppe und
- ➢ gegebenenfalls substituiertem $(C_1-C_4)$-Alkoxyalkyl.

**5.** Fluoreszenzfarbstoff der Formel (II) nach einem der vorhergehenden Ansprüche, worin Y für ein Alkalimetall oder eine unter:

- ➢ $(C_1-C_4)$Alkylcarbonyl;

- ➢ Arylcarbonyl;
- ➢ $(C_1-C_4)$ Alkoxycarbonyl;
- ➢ Aryloxycarbonyl;
- ➢ Aryl $(C_1-C_4)$ alkoxycarbonyl;
- ➢ (Di) $(C_1-C_4)$ (alkyl)aminocarbonyl;
- ➢ $(C_1-C_4)$ (Alkyl)arylaminocarbonyl;
- ➢ gegebenenfalls substituiertem Aryl;
- ➢ 5- oder 6-gliedrigem kationischem monocyclischem Heteroaryl;
- ➢ 8- bis 11-gliedrigem kationischem bicyclischem Heteroaryl;
- ➢ dem kationischen Heterocyclus der nachstehenden Formel:

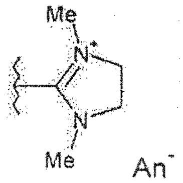

- ➢ Isothiouronium $-C(NH_2)=N^+H_2$; $An^-$;
- ➢ Isothioharnstoff $-C(NH_2)=NH$ und
- ➢ $SO_3^-$ ; $M^+$, wobei $M^+$ für ein Alkalimetall steht oder auch $M^+$ und M' der Formel (II) fehlen;

ausgewählte Schutzgruppe steht.

**6.** Fluoreszenzfarbstoff der Formel (I) oder (II) nach einem der vorhergehenden Ansprüche der folgenden Formel:

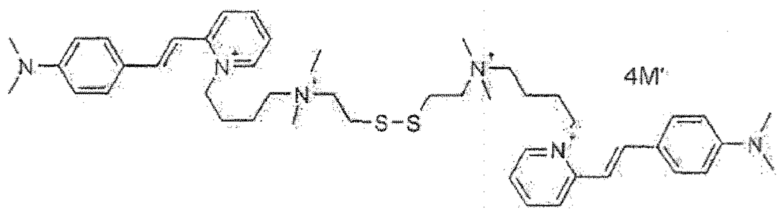

1

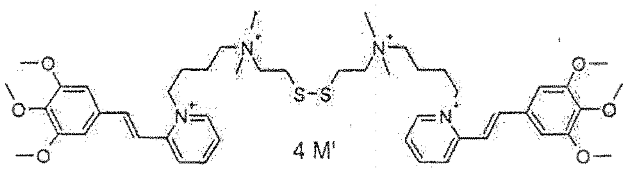

2

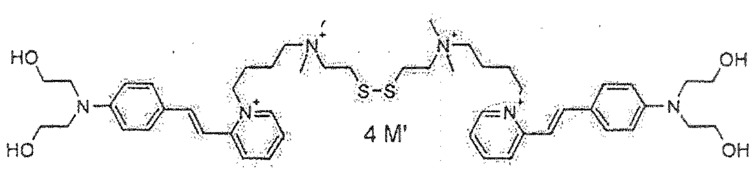

3

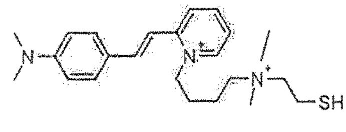
2M'

4

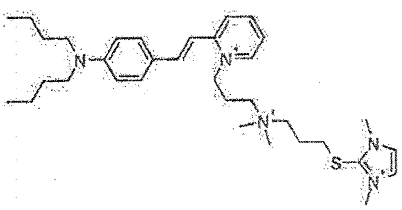
3M'

5

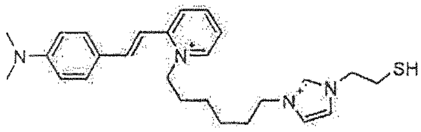
2M'

6

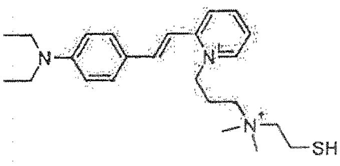
2M'

7

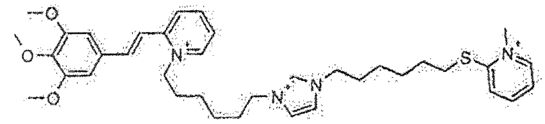
3M'

8

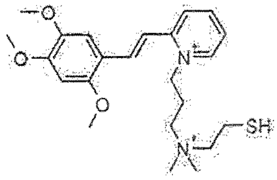
2M'

9

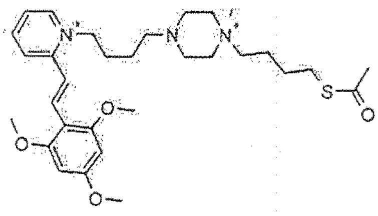
2M'

10

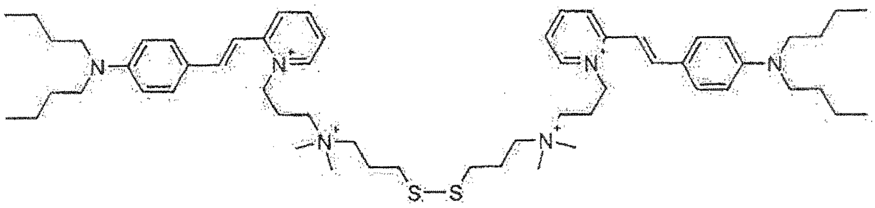
4M'

11

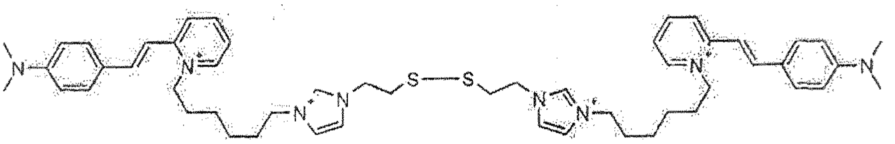
4M'

12

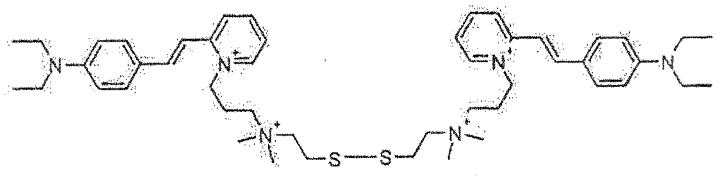

4M'

13

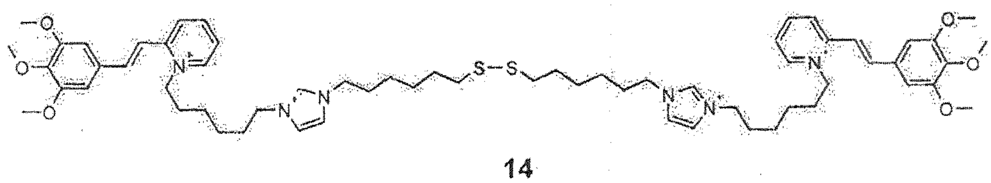

4M'

14

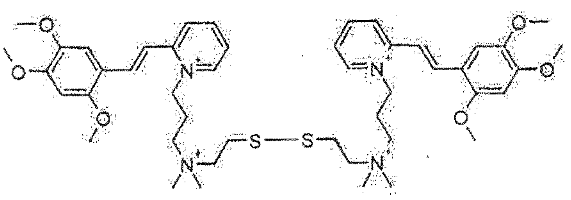

4M'

15

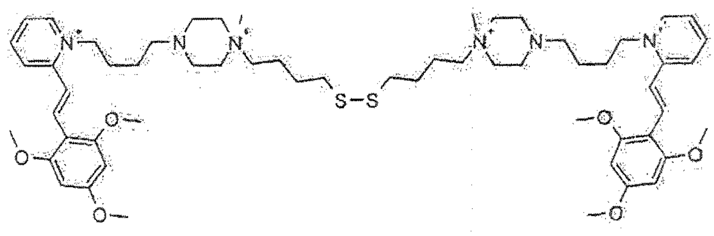

4M'

16

wobei die Gruppen M' gleich oder verschieden sind und für ein anionisches Gegenion stehen.

**7.** Färbezusammensetzung, die in einem geeigneten kosmetischen Medium einen Fluoreszenzfarbstoff der Formel (I) oder (II) gemäß einem der Ansprüche 1 bis 6 umfasst.

**8.** Färbezusammensetzung, die in einem geeigneten kosmetischen Medium mindestens einen Fluoreszenzfarbstoff der Formel (I) oder (II) gemäß einem der Ansprüche 1 bis 6 und mindestens ein Reduktionsmittel umfasst.

**9.** Zusammensetzung nach den Ansprüchen 7 oder 8, wobei der Fluoreszenzfarbstoff der Formel (I) oder (II) in einer Menge zwischen 0,001 und 50 Gew.-%, bezogen auf das Gesamtgewicht der Zusammensetzung, vorliegt.

**10.** Verfahren zum Färben von Keratinmaterialien, bei dem man auf die Materialien eine Färbezusammensetzung gemäß einem der Ansprüche 7 bis 9, die in einem geeigneten kosmetischen Medium mindestens einen Fluoreszenzfarbstoff der Formel (I) oder (II) gemäß einem der Ansprüche 1 bis 6 umfasst, aufbringt, gegebenenfalls in Gegenwart eines Reduktionsmittels, das zur Reduktion der Disulfidbindungen von Keratinmaterialien befähigt ist.

**11.** Verfahren zum Färben von Keratinmaterialien nach Anspruch 10, **dadurch gekennzeichnet, dass** es sich bei den Keratinmaterialien um dunkle Keratinfasern mit einer Tonhöhe kleiner gleich 6 handelt.

**12.** Verfahren nach den Ansprüchen 10 oder 11, bei dem man das Reduktionsmittel vor oder nach dem Aufbringen des Thiol-Fluoreszenzfarbstoffs aufbringt.

**13.** Verfahren nach einem der Ansprüche 10 bis 12 mit einem zusätzlichen Schritt, der daraus besteht, dass man auf die Keratinfasern ein Oxidationsmittel aufbringt.

**14.** Vorrichtung mit mehreren Kompartimenten, in der ein erstes Kompartiment eine Färbezusammensetzung, die einen Fluoreszenzfarbstoff gemäß einem der Ansprüche 1 bis 6 umfasst, enthält und ein zweites Kompartiment ein Reduktionsmittel, das zur Reduktion der Disulfidbindungen von Keratinmaterialien befähigt ist, enthält.

**15.** Verwendung der Fluoreszenzfarbstoffe gemäß einem der Ansprüche 1 bis 6 zum Aufhellen von dunklen Keratinfasern mit einer Tonhöhe kleiner 6.

## Claims

**1.** Fluorescent dye of following formula (I) or (II):

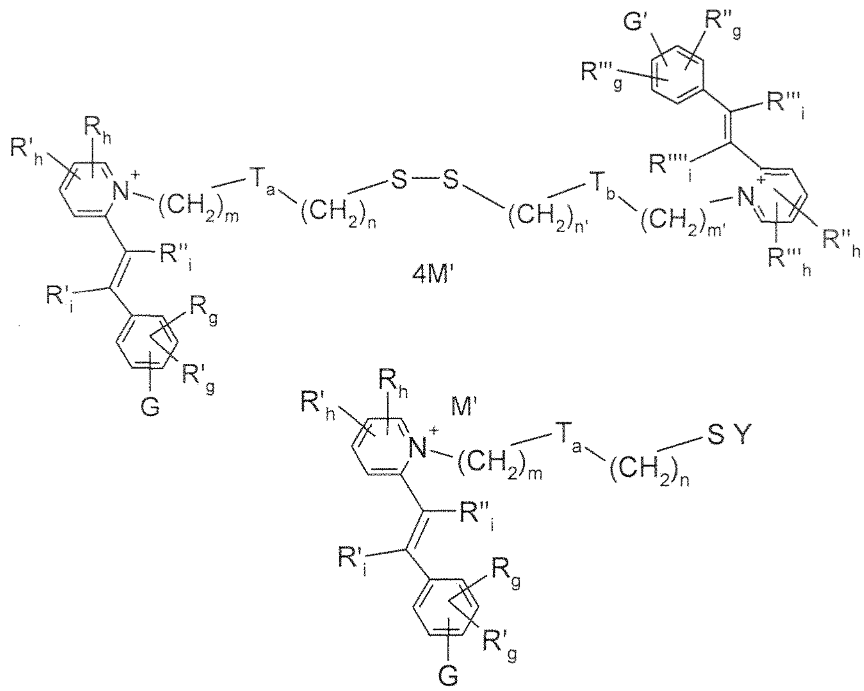

(I)

(II)

the organic or mineral acid salts, optical isomers and geometrical isomers thereof, and the solvates such as hydrates; in which formulae (I) and (II):

> ➢ G and G', which may be identical or different, represent an optionally substituted $-NR_cR_d$ or $C_1$-$C_6$ alkoxy group;
> ➢ $R_c$ and $R_d$, which may be identical or different, represent a hydrogen atom, an aryl($C_1$-$C_4$)alkyl group, or an optionally substituted ($C_1$-$C_6$)alkyl group;
> ➢ $R_g$, $R'_g$, $R''_g$, $R'''_g$, $R_h$, $R'_h$, $R''_h$ and $R'''_h$, which may be identical or different, represent a hydrogen or halogen atom, an amino, (di) ($C_1$-$C_4$) alkylamino, cyano, carboxyl, hydroxyl, trifluoromethyl, acylamino, $C_1$-$C_4$, alkoxy, $C_2$-$C_4$ (poly)hydroxyalkoxy, ($C_1$-$C_4$)alkylcarbonyloxy, ($C_1$-$C_4$) alkoxycarbonyl, ($C_1$-$C_4$)alkylcarbonylamino, acylamino, carbamoyl or ($C_1$-$C_4$)alkylsulfonylamino group, an aminosulfonyl radical, or a ($C_1$-$C_{16}$)alkyl radical optionally substituted with a group chosen from ($C_1$-$C_{12}$)alkoxy, hydroxyl, cyano, carboxyl, amino and (di)($C_1$-$C_4$) alkylamino, or else the two alkyl radicals borne by the nitrogen atom of the amino group form a heterocycle comprising 5 to 7 members and optionally comprising another heteroatom which may be identical to or different from that of the nitrogen atom;
> ➢ $R'_i$, $R''_i$, $R'''_i$ and $R''''_i$, which may be identical or different, represent a hydrogen atom or a ($C_1$-$C_4$)alkyl group;
> ➢ $T_a$ and $T_b$, which may be identical or different, represent:
>
> • either an ammonium radical $-N^+(R)(R°)-$, with R and R°, which may be identical or different, representing a hydrogen atom, a $C_1$-$C_4$ alkyl or $C_1$-$C_4$ hydroxyalkyl radical or an aryl($C_1$-$C_4$) alkyl;

• or a preferably monocyclic, cationic, heterocycloalkyl or heteroaryl radical containing especially two heteroatoms, particularly two nitrogen atoms, and comprising in particular from 5 to 7 members, such as imidazolium, pyridinium or pyrrolidinium optionally substituted with a $(C_1-C_4)$alkyl group such as methyl;

➢ m, m', n and n', which may be identical or different, represent an integer between 0 and 6 inclusive with m+n, m'+n' representing an integer between 1 and 10 inclusive;
➢ M', which may be identical or different, represents an anionic counterion; and
➢ Y represents: i) a hydrogen atom; ii) an alkali metal; iii) an alkaline earth metal; iv) an ammonium group: $N^+R^\alpha R^\beta R^\gamma R^\delta$ or a phosphonium group: $P^+R^\alpha R^\beta R^\gamma R^\delta$ with $R^\alpha$, $R^\beta$, $R^\gamma$ and $R^\delta$, which may be identical or different, representing a hydrogen atom or a $(C_1-C_4)$alkyl group; or v) a thiol-function-protecting group;

it being understood that:

- when the compound of formula (I) or (II) contains other cationic parts, it is associated with one or more anionic counterions allowing formula (I) or (II) to achieve electroneutrality.

**2.** Fluorescent dye of formula (II) according to the preceding claim, in which Y represents a hydrogen atom or an alkali metal.

**3.** Fluorescent dye of formula (II) according to Claim 1, in which Y represents a protecting group.

**4.** Fluorescent dye of formula (II) according to the preceding claim, in which Y represents a protecting group chosen from the following radicals:

➢ $(C_1-C_4)$alkylcarbonyl;
➢ $(C_1-C_4)$alkylthiocarbonyl;
➢ $(C_1-C_4)$alkoxycarbonyl;
➢ $(C_1-C_4)$alkoxythiocarbonyl;
➢ $(C_1-C_4)$alkylthiothiocarbonyl;
➢ (di) $(C_1-C_4)$ (alkyl)aminocarbonyl;
➢ (di) $(C_1-C_4)$ (alkyl)aminothiocarbonyl;
➢ arylcarbonyl;
➢ aryloxycarbonyl;
➢ aryl $(C_1-C_4)$alkoxycarbonyl;
➢ (di) $(C_1-C_4)$ (alkyl)aminocarbonyl;
➢ $(C_1-C_4)$ (alkyl)arylaminocarbonyl;
➢ carboxyl;
➢ $SO_3^-$ ; M+ with M+ representing an alkali metal or else $M^+$ and M' of formula (II) are absent;
➢ optionally substituted aryl,
➢ optionally substituted heteroaryl;
➢ optionally cationic, optionally substituted heterocycloalkyl;
➢ isothiouronium $-C(NR'^cR'^d)=N^+R'^eR'^f$; $An^-$ with $R'^c$, $R'^d$, $R'^e$ and $R'^f$, which may be identical or different, representing a hydrogen atom or an alkyl group; and An- represents an anionic counterion;
➢ isothiourea $-C(NR'^cR'^d)=NR'^e$ with $R'^c$, $R'^d$ and $R'^e$ as defined above;
➢ optionally substituted (di)arylalkyl;
➢ optionally substituted (di)heteroarylalkyl;
➢ $CR'^1R'^2R'^3$ with $R'^1$, $R'^2$ and $R'^3$, which may be identical or different, representing a halogen atom or a group chosen from:

• alkyl;
• alkoxy;
• optionally substituted aryl;
• optionally substituted heteroaryl;

➢ $P(Z^1)R''^1R''^2R''^3$ with $R''^1$ and $R''^2$, which may be identical or different, representing a hydroxyl, alkoxy or alkyl group, $R''^3$ representing a hydroxyl or alkoxy group and $Z^1$ representing an oxygen or sulfur atom;
➢ a sterically hindered cyclic group, such as the adamantyl group; and
➢ optionally substituted alkoxy$(C_1-C_4)$alkyl.

**5.** Fluorescent dye of formula (II) according to any one of the preceding claims, in which Y represents an alkali metal or a protecting group chosen from:

- ➢ $(C_1\text{-}C_4)$alkylcarbonyl;
- ➢ arylcarbonyl;
- ➢ $(C_1\text{-}C_4)$alkoxycarbonyl;
- ➢ aryloxycarbonyl;
- ➢ aryl $(C_1\text{-}C_4)$alkoxycarbonyl;
- ➢ (di) $(C_1\text{-}C_4)$ (alkyl)aminocarbonyl;
- ➢ $(C_1\text{-}C_4)$ (alkyl)arylaminocarbonyl;
- ➢ optionally substituted aryl;
- ➢ 5- or 6-membered monocyclic heteroaryl;
- ➢ 8- to 11-membered cationic bicyclic heteroaryl;
- ➢ cationic heterocycle of formula below:

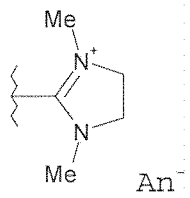

- ➢ isothiouronium $-C(NH_2)=N^+H_2$; $An^-$;
- ➢ isothiourea $-C(NH_2)=NH$; and
- ➢ $SO_3^-$ ; $M^+$ with $M^+$ representing an alkali metal or else $M^+$ and M' of formula (II) are absent.

**6.** Fluorescent dye of formula (I) or (II) according to any one of the preceding claims, of the following formula:

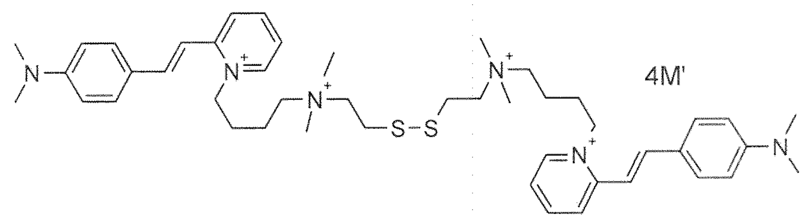

4M'

1

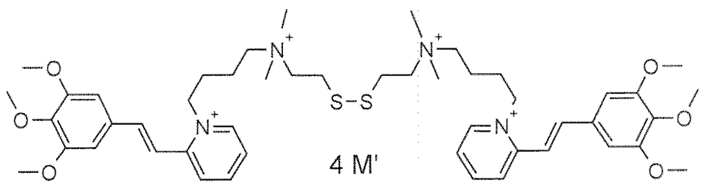

4 M'

2

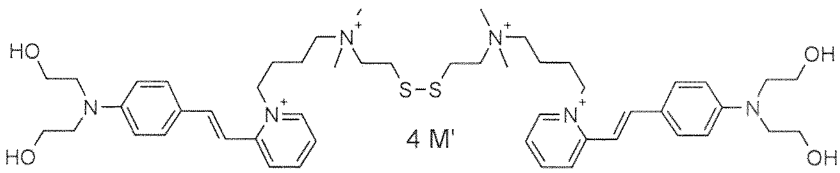

4 M'

3

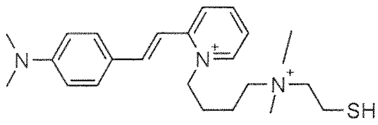

2M'

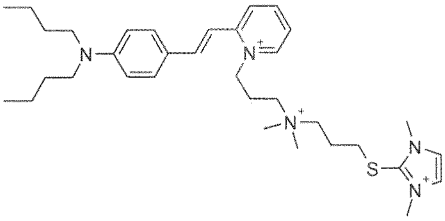

3M'

4

5

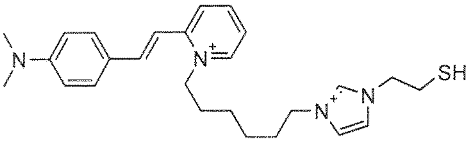

2M'

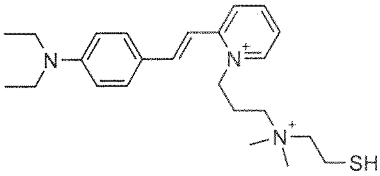

2M'

6

7

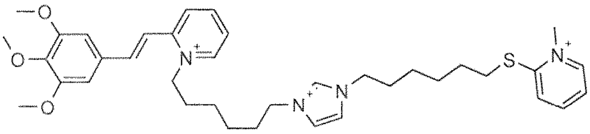

3M'

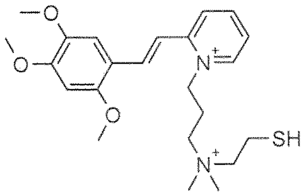

2M'

8

9

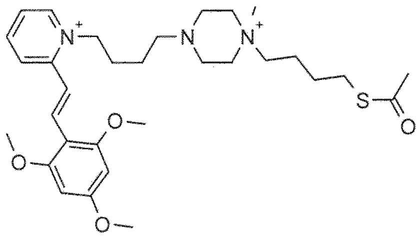

2M'

10

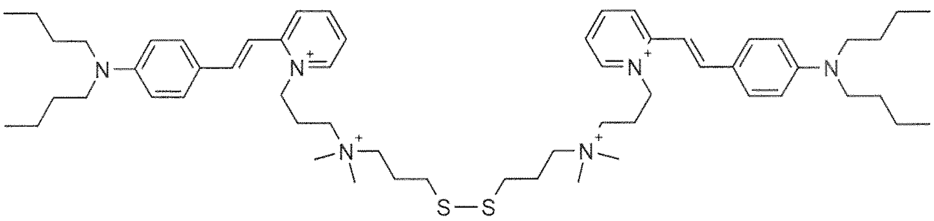

4M'

11

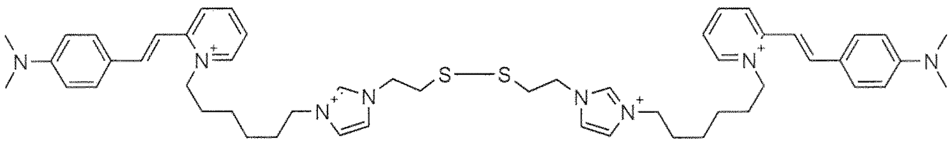

4M'

12

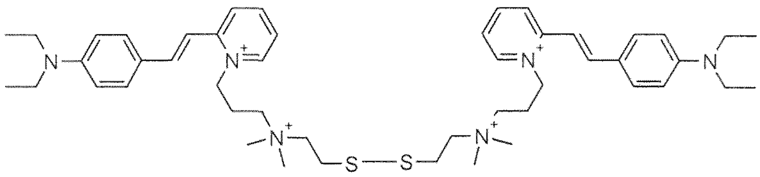

4M'

13

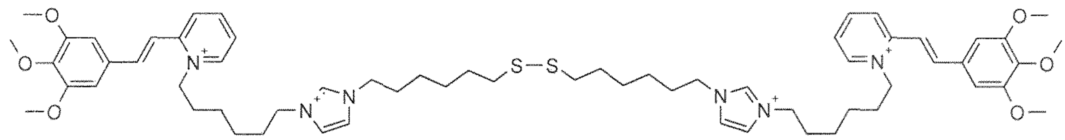

4M'

14

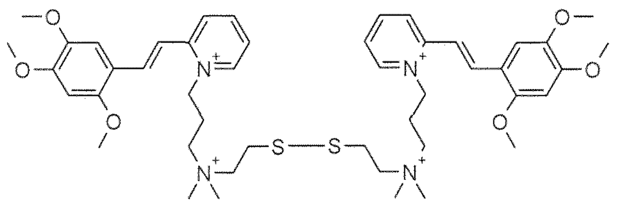

4M'

15

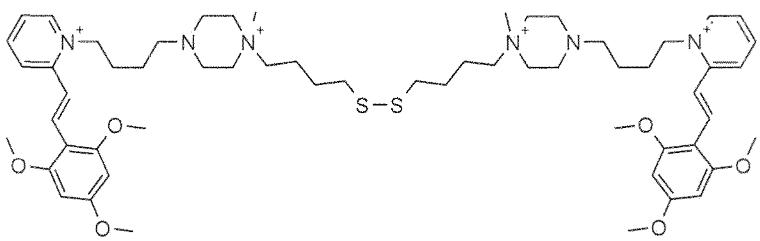

4M'

16

with M', which may be identical or different, representing an anionic counterion.

**7.** Dye composition comprising, in a suitable cosmetic medium, a fluorescent dye of formula (I) or (II) as defined in any one of Claims 1 to 6.

**8.** Dye composition comprising, in a suitable cosmetic medium, at least one fluorescent dye of formula (I) or (II) as defined in any one of Claims 1 to 6 and at least one reducing agent.

**9.** Composition according to Claim 7 or 8, in which the fluorescent dye of formula (I) or (II) is present in an amount of between 0.001% and 50% by weight, relative to the total weight of the composition.

**10.** Process for dyeing keratin materials, in which a dye composition as defined in any one of Claims 7 to 9, comprising, in a suitable cosmetic medium, at least one fluorescent dye of formula (I) or (II) as defined in any one of Claims 1 to 6, optionally in the presence of a reducing agent capable of reducing the disulfide bonds of the keratin materials, is applied to the materials.

**11.** Process for dyeing keratin materials according to Claim 10, **characterized in that** the keratin materials are dark keratin fibres having a tone height of less than or equal to 6.

**12.** Process according to Claim 10 or 11, in which the reducing agent is applied before or after the application of the thiol fluorescent dye.

**13.** Process according to any one of Claims 10 to 12, comprising an additional step consisting in applying an oxidizing agent to the keratin fibres.

**14.** Multicompartment device in which a first compartment contains a dye composition comprising a fluorescent dye as defined in Claims 1 to 6 and a second compartment contains a reducing agent capable of reducing the disulfide bonds of keratin materials.

**15.** Use of the fluorescent dyes as defined in Claims 1 to 6, for lightening dark keratin fibres which have a tone height of less than 6.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2830189
- WO 2004091473 A
- CA 2024509
- WO 9951194 A
- FR 1156407
- WO 2005097051 A
- WO 02078596 A
- WO 2005004822 A
- US 6262263 B1
- FR 2586913


### Littérature non-brevet citée dans la description


- **GUISE ; STAPLETON.** *Journal of the Society of Dyers and Colourists,* 1975, vol. 91, 259-264
- *Journal of Cosmetic Chemistry,* 1991, vol. 42, 1-17
- *Mol. Cryst. Liq. Cryst.,* vol. 377, 137-140
- **DE CHARLES ZVIAK.** Science des traitements capillaires. 1988, 215, 278
- **T. W. GREENE.** Protective Groups in Organic Synthesis. John Willey & Sons, 1981, 193-217
- **P. KOCIENSKI.** Protecting Groups. Thieme, 2005
- « Thiols and organic Sulfides », « Thiocyanates and Isothiocyanates, organic ». Ullmann's Encyclopedia. Wiley-VCH, 2005
- Reactions, Mechanisms and Structures. **J. MARCH.** Advanced Organic Chemistry. John Willey & Sons, 1992
- **T. W. GREENE.** Protective Groups in Organic Synthesis
- **T. W. GREENE.** Protective Groups in Organic Synthesis. John Willey & Sons, 1981